# FASCICULE DE BREVET EUROPEEN

(11) **EP 1 090 122 B1**
(45) Date de publication et mention de la délivrance du brevet: **16.07.2008**
(21) Numéro de dépôt: 99926538.2
(22) Date de dépôt: 23.06.1999
(51) Int. Cl.: C12N 15/48, C12Q 1/70, C07K 14/15, A61K 31/70

(54) **FAMILLE DE SEQUENCES NUCLEIQUES ET DE SEQUENCES PROTEIQUES DEDUITES PRESENTANT DES MOTIFS RETROVIRAUX ENDOGENES HUMAINS, ET LEURS APPLICATIONS**
NUKLEINSÄURESEQUENZFAMILIE UND ENTSPRECHENDE PROTEINSEQUENZEN, DIE HUMANE ENDOGENE RETROVIRALE MOTIVE ENTHALTEN, UND IHRE ANWENDUNGEN
NUCLEIC SEQUENCE AND DEDUCED PROTEIN SEQUENCE FAMILY WITH HUMAN ENDOGENOUS RETROVIRAL MOTIFS, AND THEIR USES

(30) Priorité: 23.06.1998 FR 9807920
(43) Date de publication de la demande: 11.04.2001
(60) Demande divisionnaire: 08010138.9
(73) Titulaire: INSTITUT NATIONAL DE LA SANTE ET DE LA RECHERCHE MEDICALE (INSERM), 75654 Paris Cédex 13 (FR)
(72) Inventeur: ALLIEL, Patrick, M., F-92140 Clamart (FR); PERIN, Jean-Pierre, F-92350 Le Plessis-Robinson (FR); RIEGER, François, F-92100 Boulogne (FR)
(74) Mandataire: Vialle-Presles, Marie José
(86) Numéro de dépôt international: PCT/FR1999/001513
(87) Numéro de publication internationale: WO 1999/067395

(56) Documents cités:
- WO-A-98/23755
- WO-A-99/02666
- WO-A-99/02696
- WO-A-99/26972
- FR-A- 2 737 500
- US-A- 5 708 157
- Database GenBank. Séquence HSAC 000064 Clone humain BAC RG083M05 de 7q21-7q22, séquence complet. 17 novembre 1996. XP002118730
- ALLIEL PM ET AL: "Séquences rétrovirales endogènes anlogues à celle du nouveau rétrovirus MSRV associé à la sclérose en plaques ( première partie)" COMPTES RENDUS DES SEANCES DE L'ACADEMIE DES SCIENCES SERIE III: SCIENCES DE LA VIE., vol. 321, no. 6, juin 1998 (1998-06), pages 495-499, XP002101380 MONTREUIL FR
- Database GenBank Séquence AC X93499 mRNA de H. sapiens pour la protéine rab7 10 février 1997 XP002119234 & VITELLI R ET AL: "Molecular cloning and expression analysis of the human rab7 GTP-ase complementary deoxyribonucleic acid" BIOCHEMICAL AND BIOPHYSICAL RESEARCH COMMUNICATIONS, vol. 229, no. 3, 1996, pages 887-890, ORLANDO, FL US
- ALLIEL PM ET AL: "Rétrovirus endogènes et sclérose en plaques. II. HERV-7q" COMPTES RENDUS DES SEANCES DE L'ACADEMIE DES SCIENCES SERIE III: SCIENCES DE LA VIE., vol. 321, no. 10, octobre 1998 (1998-10), pages 857-863, XP002101381 MONTREUIL FR
- MITANI M ET AL: "Suppressive effect on polyclonal B-cell activation of a synthetic peptide homologous to a transmembrane component of oncogenic retrovirus" PROCEEDINGS OF THE NATIONAL ACADEMY OF SCIENCES OF USA, vol. 84, no. 1, janvier 1987 (1987-01), pages 237-240, XP002118729 WASHINGTON US cité dans la demande
- RASMUSSEN H.B. ET AL ACTA NEUROL. SCAND. vol. 169, no. SUPPLEMENT, 1997, pages 32 - 37, XP002930427
- PATIENCE C. ET AL TRENDS IN GENETICS vol. 13, no. 3, Mars 1997, pages 116 - 120
- ANTONY M. ET AL NATURE NEUROSCIENCE vol. 7, no. 10, Octobre 2004, pages 1088 - 1095
- DATABASE EMBL [en ligne] 01 Février 1997 'yv43e03.r1 Soares fetal liver spleen 1NFLS Homo sapiens cDNA clone' Retrieved from EBI Database accession no. N77302

### LISTE DE SEQUENCES

<110> INSERM
   ALLIEL, Patrick
   PERIN, jean-pierre
   RIEGER, Francois
<120> SEQUENCE NUCLEIQUE ET SEQUENCE
   PROTEIQUE DEDUITE PRESENTANT UN MOTIF RETROVIRAL
   ENDOGENE HUMAIN DE TYPE ENV ET SES
   APPLICATIONS.
<130> 598EXT21
<140>
   <141>
<150> 9807920
   <151> 1998-06-23
<160> 84
<170> PatentIn Ver. 2.1
<210> 1
   <211> 2599
   <212> ADN
   <213> Homo sapiens
<400> 1
   atcccctgcc ttaatcgcca agctccttca ggagaacaaa gaacaggcca ttaccctgga 60
   gaagactggc aactgatttt acccacaagc ccaaacctca gggatttcag tatctactag 120
   tctgggtaga tactttcacg ggttgggcag aggccttccc ctgtaggaca gaaaaggccc 180
   aagaggtaat aaaggcacta gttcatgaaa taattcccag attcggactt ccccgaggct 240
   tacagagtga caatagccct gctttccagg ccacagtaac ccagggagta tcccaggcgt 300
   taggtatacg atatcactta cactgcgcct gaaggccaca gtcctcaggg aaggtcgaga 360
   aaatgaatga aacactcaaa ggacatctaa aaaagcaaac ccaggaaacc cacctcacat 420
   ggcctgctct gttgcctata gccttaaaaa gaatctgcaa ctttccccaa aaagcaggac 480
   ttagcccata cgaaatgctg tatggaaggc ccttcataac caatgacctt gtgcttgacc 540
   caagacagcc aacttagttg cagacatcac ctccttagcc aaatatcaac aagttcttaa 600
   aacattacaa ggaacctatc cctgagaaga gggaaaagaa ctattccacc cttgtgacat 660
   ggtattagtc aagtcccttc cctctaattc cccatcccta gatacatcct gggaaggacc 720
   ctacccagtc attttatcta ccccaactgc ggttaaagtg gctggagtgg agtcttggat 780
   acatcacact tgagtcaaat cctggatact gccaaaggaa cctgaaaatc caggagacaa 840
   cgctagctat tcctgtgaac ctctagagga tttgcgcctg ctcttcaaac aacaaccagg 900
   aggaaagtaa ctaaaatcat aaatccccat ggccctccct tatcatattt ttctctttac 960
   tgttctttta ccctctttca ctctcactgc accccctcca tgccgctgta tgaccagtag 1020
   ctccccttac caagagtttc tatggagaat gcagcgtccc ggaaatattg atgccccatc 1080
   gtataggagt ctttctaagg gaacccccac cttcactgcc cacacccata tgccccgcaa 1140
   ctgctatcac tctgccactc tttgcatgca tgcaaatact cattattgga caggaaaaat 1200
   gattaatcct agttgtcctg gaggacttgg agtcactgtc tgttggactt acttcaccca 1260
   aactggtatg tctgatgggg gtggagttca agatcaggca agagaaaaac atgtaaaaga 1320
   agtaatctcc caactcaccc gggtacatgg cacctctagc ccctacaaag gactagatct 1380
   ctcaaaacta catgaaaccc tccgtaccca tactcgcctg gtaagcctat ttaataccac 1440
   cctcactggg ctccatgagg tctcggccca aaaccctact aactgttgga tatgcctccc 1500
   cctgaacttc aggccatatg tttcaatccc tgtacctgaa caatggaaca acttcagcac 1560
   agaaataaac accacttccg ttttagtagg acctcttgtt tccaatctgg aaataaccca 1620
   tacctcaaac ctcacctgtg taaaatttag caatactaca tacacaacca actcccaatg 1680
   catcaggtgg gtaactcctc ccacacaaat agtctgccta ccctcaggaa tattttttgt 1740
   ctgtggtacc tcagcctatc gttgtttgaa tggctcttca gaatctatgt gcttcctctc 1800
   attcttagtg ccccctatga ccatctacac tgaacaagat ttatacagtt atgtcatatc 1860
   taagccccgc aacaaaagag tacccattct tccttttgtt ataggagcag gagtgctagg 1920
   tgcactaggt actggcattg gcggtatcac aacctctact cagttctact acaaactatc 1980
   tcaagaacta aatggggaca tggaacgggt cgccgactcc ctggtcacct tgcaagatca 2040
   acttaactcc ctagcagcag tagtccttca aaatcgaaga gctttagact tgctaaccgc 2100
   tgaaagaggg ggaacctgtt tatttttagg ggaagaatgc tgttattatg ttaatcaatc 2160
   cggaatcgtc actgagaaag ttaaagaaat tcgagatcga atacaacgta gagcagagga 2220
   gcttcgaaac actggaccct ggggcctcct cagccaatgg atgccctgga ttctcccctt 2280
   cttaggacct ctagcagcta taatattgct actcctcttt ggaccctgta tctttaacct 2340
   ccttgttaac tttgtctctt ccagaatcga agctgtaaaa ctacaaatgg agcccaagat 2400
   gcagtccaag actaagatct accgcagacc cctggaccgg cctgctagcc cacgatctga 2460
   tgttaatgac atcaaaggca cccctcctga ggaaatctca gctgcacaac ctctactacg 2520
   ccccaattca gcaggaagca gttagagcgg tctcggccaa cctccccaac agcacttagg 2580
   ttttcctgtt gagatgggg                                                2599
<210> 2
   <211> 10499
   <212> ADN
   <213> Homo sapiens
<400> 2
<210> 3
   <211> 2784
   <212> ADN
   <213> Homo sapiens
<400> 3
   ctccttcagg agaacaaaga acaggccact acccaagaga agactggcaa ctagatttta 60
   cccatatgcc caaatctcag ggatttcagt atctactagt ttgggtagat actttcactg 120
   gttgggcaga ggccttcccc tgtaggacag aaaaggccca agaggtaata aacgttcatg 180
   aaataattcc cagattcgga cttccccaag gcttacagag tgacaatggc cctgctttca 240
   aggctacagt aacccaagga gtatcccagg tgttaggtat acaatatcac tcacactgcg 300
   cctggaggcc acagtcctca ggaaaggtgg agaaaatgaa caaaacactc aaatgacatc 360
   taaaaaagct aatccaggaa acccacctcg catggcctgc tctgttgcct atagccttac 420
   taagaatccg aaactctccc caaaaagcag gacttagtcc atacaaaatg ctgtatggac 480
   ggcccttcct aaccaatgaa cttgggcttg accgagagac agccaactta gttgcagaca 540
   tcatctcctt agccaaatat caacaggttc ttaaaacatt acagggagcc tgtccccaag 600
   aagagggaaa ggaactattc caccctggtg acatggtatt agtcaagtcc cttccctcta 660
   attccccatc cctagataca tcctgggaag gaaactaccc agccatttta tctaccctaa 720
   cggcagttaa agtggctgga gcggagtctt ggatacatca cactcaagtc aaaccctgga 780
   tactgccaaa ggaactcaaa aatccatgag acaatgctag ctattcctgt gaacctctag 840
   aggatctgcg cctgctcttc aaatgacaac cagggggaaa gtaactaaaa tcgtaaatcc 900
   cctggccctc ccttatcata tttttctctt tactgttctc ttaccccctt tcactctcac 960
   tgcaccccgt ccatgccact gcaccccgtc catgccccgt ccatgccagt agctcccctt 1020
   agcaagagtt tctatggaga atgcagcgtc ccggaaatat tgatgcccca ttgtatagga 1080
   gtttatctaa gggaaccccc accttcactg cccacaccca tatgccccac aactgctata 1140
   actctgccac tctttgcatg catgcaaata ctcattattg gacaggaaaa acgattaatc 1200
   ccagttgtcc tggaggactt ggaggactca cttcactcat accagtatgt ctgatggggg 1260
   tggagttcaa gatcaggcaa cagaaaaaca cataaaggaa gtaatctccc aactgacctg 1320
   ggtacatagc acccctggcc cctacaaagg actagatctc tcaaaactac atgaaaccct 1380
   ccatacccat actggcctgg taagcctatt taataccacc ctgactgggc tccatgaggt 1440
   ctcggcccaa aaccctacta actgttggat gtgcctcccc ctgcacttta ggccatacat 1500
   ttcaatccct atacctgaac aatggaacaa cttcagcaca gaaataaaca ccacttctgt 1560
   tttagtaggt cctctttcca atctggaaat aacccatacc tcaaacctca cctgtgtaaa 1620
   atttagcaat actatagaca cagccaactc ccaatgcatc aggtgggtaa ctcctcccac 1680
   acgaatagtc tgcctaccct caggaatatt ttttgtctgt ggtacctcag cctatcattg 1740
   tttgaatggc tcttcagaat ctgtgtgctt cctctcattc ttagtggccc ctatgcccat 1800
   ctacactgaa caagatttat acaatcatgt catacctaag ccccgcaaca aaagagtacc 1860
   cattcttcct tttgttattg gagcaggagt gctaggcgga gtagctactg gcattggcgg 1920
   tatcacaacc tctactcagt tctactacaa actgtctcaa gaactaaatg gtgacatgga 1980
   atgggtcgct gataccctgg tcaccttgca agatcaactt aactccctag cagcagtagt 2040
   ccttcaaaat cgaagagctt tagacttgct aaccgcggaa agcgggggaa cctttttatt 2100
   tttagaggaa aaatgctgtt gttatgttaa tcaatccgga atcatcaccg agaaagttaa 2160
   agaaattcaa ggtcgaatat aacgtagagc aaaggagctg caaaacactg gaccctgggg 2220
   cctcctcagc caatggatgc cctggattct ccccttctta ggacctctag cagctataat 2280
   attgttactc ctctttggac cctgtatctt taacctcctt gttaagtttg tcttttccag 2340
   aatcgaagca gtaaaactac aaatcgttct tcaaatggag ccccagatgc agtccatgag 2400
   taaaatctac cacggacccc tggaccggcc tgctagccca tgctctgatg ttaatgacat 2460
   caaaggcacc cctcccgagg aaatctcaac tgcacaacct ctactacgcc ccaattcagc 2520
   aggaagcagt tagagtggtt gttggccaac ctccccaaca gcagttgggt tttcctgttg 2580
   agagggggga ctgagagaca ggaataacta gatttcctag accaactaag aatccctaag 2640
   actagctggg aaggtgaccg cttccacctt taaacaccgg gcttgcaact tagctcacgc 2700
   ccaaccaatc agatactaaa gagagctcac taaaatgcta attaggcaaa aacaggagat 2760
   aaagaaatag ccaatcatct gttg                                        2784
<210> 4
   <211> 1799
   <212> ADN
   <213> Homo sapiens
<400> 4
   gggattctta gtcggcctag gaaatccagc taatcctgtc tctcagtccc cccactcaac 60
   aggaaaaccc aagtgctgtt ggggaggttg gctgacgacc agtctaactg cttcctgcgg 120
   aattggggca tagtaggggt tgtgcagttg agatttcctc gggaggggtg cgttcgatat 180
   cattacaatt ggagcatggg ctagtaggcc ggtccagggg tccacggtag atcttagtca 240
   tggacttcat ctggggttcc atttgaagaa cgatttgtag ctttacaact ttgattctgg 300
   aagagacaaa cttaacaagg aggttaaaga tacagggtcc aaagaggagt atcaatatta 360
   gagctgctag agatcctaag aaggggagaa tccagggcat ccattggctg aggaggcccc 420
   agggtctggt gtttttgaag ctcctctgtt ctacgttgta ttcaatctcg aatttcttca 480
   actttctctg tgacaattca ggattgatta acataataac aacattcttc cgctaaaata 540
   acataataac aacattcttc ccctaaaaat aaacagcttc cccctctttc agaggttagc 600
   aagtctaaag ctcttcaatt ttgaaggact actgatgcta ggaagttaag ttgatcttgc 660
   aaggtgacca gggagtcggc aacccattcc atgtcaccat tgagttcttg agatagtttg 720
   tagtagaact gagtagaggt tgtggtaccg ccaatgccag aacctagtcc acctagcact 780
   cctgctccga taacaaaagg aagaatgagt actcttttgt tgtggggctt aggtacaaca 840
   taattgtata aatcttgttc agtgtaaatg gtcatggggg cactaagaat gagaggaagc 900
   acatagattc tgaagagcca ttcaaacaac gataggctaa ggtaccacag acaaaaaata 960
   ttcctgaggg taggcagact attcgtgtgg gaggagttac ccacctgatg cattgggagt 1020
   tggttgtgtc tacagtattg ctaaatttta cacaggtgag gtttgaggta tgggttattt 1080
   ccagattgga aacaagaggt cctactaaaa cggaagtggt gtttatttct gtgctgtagt 1140
   tgttccattg ttcaggtaca gggattgaaa tgcatggcct gaaatacagg gggaggcaca 1200
   accaacagtt agtagggttt tggaccgaga cctcatggag cccagtgagg gtggtattaa 1260
   ataggcttac caggcaagta tgggtatgga gggtttcatg tagttttaag agatctagtc 1320
   ctttgtaggg gctaggggtg ctatgtaccc gggtcagttg ggaggttact tcctttacat 1380
   gtttttctct tgcctgatct tgaactccac ccccctcaga cataccagta tgggtgaagt 1440
   aagtccgaca gacagtggct ccaagtcttc caggacaact aggattaatc attttccctg 1500
   tccaataatg agtatttgca tgcatgcaaa gagtggcaga gttatagcag ttgtggggca 1560
   tatgggtgtg ggcagtgaag gtggagtttc ctttaggtaa actcctattt gatggggcat 1620
   caatatttct gggaagccgc attcttcata gaaactcttg gtaaggggag ctgctggttg 1680
   tacagcagca tggagggggt gcagtgagag tgaaaggggg taagagaaca gtaaagagaa 1740
   aaatatgata agggagggcc atggggattt acgattttag ttactttcct cacggttgt  1799
<210> 5
   <211> 1216
   <212> ADN
   <213> Homo sapiens
<400> 5
   taaagataca gggattgaaa tgtatggcct gaagtgcagg gtcatatagg tgtgggtggt 60
   gaaaatgggg tttcctttag aaaaactcct atacgatggg tcatcaatat ttccaggaag 120
   ccgcattctc catagaagct cttggtaatg ggagctactg gtagtacagt ggcatggagg 180
   gggtgcagtg agagtgaaag agggtaaaag aacagtaaag agaaaaatat gataagggag 240
   gggttcagtg agagtgaaag ggggtaagag aacagtaaag aaaaaaatat gacaaggagg 300
   gccatgagga tctacgattc tagttacttt cctcacggtt gtcgcttgaa gagcaggtgc 360
   agatcctcta gaggttcaca ggaatagcta gcgttgtctc ctggattttc gggttccttt 420
   ggcagtatac agagtttgac tcgagtgtga tgtattcaag actccactcc agccacttta 480
   accgcagttg gggtagataa aatgactggg tagggtcctt cccaggatgt atctaaggat 540
   ggggacttag aaggaaggga cttgactaat accatgtcac cagggtgcaa taattacttt 600
   ccctcttctc gggaacaggt tccctgtaat gttttaagaa cttgttgata tttggccaag 660
   gaggtgatgt ctgcaactaa gctggccatc tctcggtcaa gcacaaggtc cttggttagg 720
   aagggccatc catacagcat tttgtatggg ctaagtcctg ctttttgggg agagttttgg 780
   attcttagta aggctgtagg caacagagca ggccatgcaa ggtgggtttc ttgggttagc 840
   ttttttaaat gtcgtttgag tgcttcattc attttcttga cttttcctga ggattgtggc 900
   ctccacgcgc agtgtaagtg atattgtatg cctaatgcct gggatactcc ctgggttact 960
   gtagccttga aaacggggcc attgtcactc tgtaagcctc ggggaagtcc gaatctggga 1020
   attatttcat gaattagtgc ctttattaca tcttggtcct tttctgtcct acaaaggaag 1080
   gcctctgccc aaccagtgaa aatatctacc cagactagta gatactgaaa tccctgagat 1140
   ttgggcatgt gggtaaaatc tagttgccag tcttctcctg agtaatggcc tgttctttgt 1200
   tctcctgaag gagctt                                                 1216
<210> 6
   <211> 976
   <212> ADN
   <213> Homo sapiens
<400> 6
   agtgataatg gaatacttga aagtaatccc ctcactccag gaactagtgc tgagctggcc 60
   aaactaatag ccctcactcg ggcactagaa ttaggagaag agaaaagggt aaatatatat 120
   acagactata agtatgctta cctagtcctt catgcccatg cagcaatatg gagagaaagg 180
   gaattcctaa cttccaaagg aacacctatc aaacatcagg aagccattag gatattatta 240
   ttggtggtac agaaacctaa agaggtggca gtcctacact gctggggtca tcagaaaaaa 300
   aaggaaaggg aaatagaagg gaactaccaa gcagatattg aagccaaaag agccgcaagg 360
   caggaccctc cattagaaat gcttatagaa ggacccctag tgtggggtaa ccccctccag 420
   gaaagcaatc cccagtactc agcaggagaa ataaaatgga gaacctcacg aggacatact 480
   ttcctcccct caggatggct agccaccaaa gaaggaaaaa tgcttttgcc tgcagctaac 540
   caatggaaat tacttaaaac ccttcaccaa acctttcact taggattgat agcacccatc 600
   agatggccaa attattattt actggatcag gccttttcaa aactatcaag caggtagtca 660
   gggcctgtaa agtgtgccaa agaaataatc tcctgcactg caagccatac atttcaatcc 720
   ctgtatcttt aacctccttg ttaagtttgt ctcttccaga atcaaagctg taaaactaca 780
   aatggttctt caaatggagt ctcagatgca gtccatgact aagatatacc gcagccccct 840
   ggagggggcc tgctagccca tgctccaatg ttaatgacat cgaaggcacc cctcccgggg 900
   aaatctcaac tgcacaaccc ctactatgtc ccaattcagc aggaagcagt taaagcggtc 960
   atcggccaac ctcccc 976
<210> 7
   <211> 942
   <212> ADN
   <213> Homo sapiens
<400> 7
   agaggagaac agcagcataa gcggctggca gaggtaggga aagaccagca agaagaaaag 60
   agagaaagag aaagagaaag tcagagaaag agacagagag aggaagagac aaagagacag 120
   aaagtcaaag aggtagtagt cagaaacaga gacaaaaaaa aggagtcaga aagagggaca 180
   gacacagaaa gtcaaaaaaa aagttaagaa gaaaggaaaa gacaaagaag aagtcgaaga 240
   ggagaaagag agagatagaa gtagtaaaga aaaaaacagc atatcccatt cctttaaagc 300
   cagggtaaat ttctatctac ccagccaagg catattctac ttatgtggat cttcaaccca 360
   tatctgcctc tcagacagtt tgcaagaaat aatgaaatct atccttactt tacaatccca 420
   aatagactct ttggcagcag tgactctcca aaactgcaga ggcctagacc tcctcactgc 480
   tgaaaaagga ggacactaca ccttcttagg ggaagaatgt tgtttttaca ctaaccagtc 540
   ggggatagta tgagatgctg cccggagttt acaggaaaag gcttctgaaa tcagacaacg 600
   cctttcaaat tcttatacca acttctggag ttaggcaaca tggcttctcc cctttctagg 660
   tcctgtggca gccatcttgc tgttactcgc ctttgggccc tgtattttta accttcttgt 720
   caaatttgtt tcctctagaa tcgaggccat caagctacag atggtcttac aaatggaacc 780
   ccaaaagagt tcaactaaca acttctaccg aggacccctg gatcaaccca ctggcacttc 840
   ccctggccta gagagttccc ctctgaagga caccgcaact gcagggccct tctttgcccc 900
   atccagcagg agtagctaga gtggtcatcg gccaaattgc ca 942
<210> 8
   <211> 944
   <212> ADN
   <213> Homo sapiens
<400> 8
   acctaggagg aactgtcttc aggacaggac tatagatgct tcctcccagg cgattaaggg 60
   aaaaagacac aatgggtatt cagtaagtga taaggaaact cttgtagaag cagagttagg 120
   aaaattgcct aataattggt ctgctcaaat gtgcgagctg tttgcactca gccaaacctt 180
   aaaagtatta cagaatcagg aagaagccat ctataccaat tctaagttaa tatggactga 240
   acgagaactt attaatagca aagaataatt gaaatcccaa acttacaagg ttttcaacaa 300
   aagcacagtt tgctaaaagt taactgtgta acatgtatta tcctactacc acaaactctc 360
   aaatgatttc tcagacagtt tgcaagaaac aatgaaacct atccttactc tacaatccca 420
   aatagactct ttggcagcag tgactctcca aaaccaccaa ggcctagacc tcctcactgc 480
   tgagaaagga ggactctgca ccttcttagg ggaagattgt tgtttttaca ctaaccagtc 540
   agggatagtg tgagatgcca cccagcgttt acaggaaaag gcttctgaaa tcagacacaa 600
   tgcttttcaa accttatagc aacctctgga gttcggcgac tggcttttcc cctttctagg 660
   tcctgtgaca gccatcttgc tattactcgc cttcgggccc tgtattttta acctcctcgt 720
   caaatttgtt tcctctagga tcgaggccat caagctacag atggtcttac aaatggaacc 780
   ccaaatgagc tcgactaaca acttctactg aggacccctg gaccgaccca ctggcccttt 840
   aactggctta aagagtttcc ctctggagga cactacaact gcagggcccc ttctttgccc 900
   catccacagg aagttagcta gagcagtcat cacccaattc ccaa                  944
<210> 9
   <211> 963
   <212> ADN
   <213> Homo sapiens
<400> 9
   tacaggaacc ccataatacg tccttggcaa attctattca gctccaactg ctaggagtgg 60
   cccatttgtc ctgaaccctc aaatcatggg aatgagaaat gaatttagac tgaccacagc 120
   ccttatgagt tttcagctac aggggtgtat agaaccctga taaggagttt tctttgtgtg 180
   tggaagatcc ttctatattt gcctccccac caactggaca ggaacttgta ctttagccta 240
   catagtacct cctgtgactt atccttttca gaagaggcag tagctgtgcc cattcatgct 300
   aagcttcagc cgagagcaat ctcactactt cctctattgg ctggtttagg atttactacc 360
   acctaggaag tggactcaca gcctagatga aatctctctc caacttactc aaatccagga 420
   ccaaatagac tcattagcag ctgtggttct ccgaaccagt gagcactaga tctccaatct 480
   cctcactgcc gaaaggggag gaacatgcct ttttctgaac aaggaatgtt gtttttatgt 540
   caataaatca ggcatagtga gagatggaat taaatgactt caggatagag ctagcagact 600
   acatggtggg acaaccgaaa ctacctcagg gttctcacag cctgttctcc actggcttct 660
   tccattttta ggtcccttcc ttatgattat tctaggagta acctttggcc catgtctttt 720
   cagttccttc atcctttcgt ttcttcctga atagaatcaa tgaaactaga aatgttactg 780
   cagatggaac ctcagatgac ttcaaccagc acctattatc aaggacccct aaaccagcct 840
   gccggcccat acccggacgt tgacacccaa accacctctc acgaggaaac ctcagctaca 900
   gaaccccttc tatgccccta ttcagcagga agcaattaga gtggtcatcc tcccacaccc 960
   caa                                                               963
<210> 10
   <211> 945
   <212> ADN
   <213> Homo sapiens
<400> 10
   ttgcagatca atctcagact gctgtgctag caatgagtga ggcttcgtgg gcatgggacc 60
   ctctgagcca ggcatgggat ataatgtcct tgtgtgccat ttgctaagac tgttggaata 120
   gcacagtatt agggtgggag tggcccgatt ttccaggtgc tgtctgtcac cgcttccctt 180
   ggctaggaaa gagaattccc tgaccccttg ttcttcccag gtaaggcagt gcctcaccct 240
   gcttcagctc acactcaggt gactgcaccc actgtcctgc ccccactgtc ggacaagccc 300
   cagtgagatg aacctggtac ctcagttgga aatgcagaaa tcacctgtct tctgcgtcac 360
   tcacactggg agctgtagac tggagctgtt cctatttggc catcttggaa ccatctccca 420
   aatagactct ttggcagcag tgactctcca aaaccaccaa ggcctagacc tcctcattgc 480
   tgagaaagga ggactctgca ccttcttagg ggaggagtgt tgtttttata ctgaccagtc 540
   agggatggta cgagatgcca cccgatgttt acaggaaaag gcttctgaaa tcacacaaca 600
   cctttcaaac tcttatacca acctctggag ttgggcaaca tggcttctcc cctttctcgg 660
   tcccattgca gccatcttgc tattactcgc cttcaggctg tgtattttta acctccttgt 720
   caaatttgtt tcctctagaa ttgaggccgt caagctacag atggtcttac aaatgggacc 780
   ccaaatgagc tcaactaaca acttctgcca aggacccctg gaccaacctg ctggcccttt 840
   cactggcctt aagagttccc ctctggaggg cactacaact gcagggcccc ttctttgccc 900
   ctatccagca ggaagtagct agagcagtca tcacccaatt cccaa                 945
<210> 11
   <211> 939
   <212> ADN
   <213> Homo sapiens
<400> 11
   agagctacct tggcaagtac tctaggagta tgggaaaatg aaaacaacaa actcacacac 60
   cattttaaca tacacaatca ggtctgccca cccagcaagg tatattcttt gtatgtggaa 120
   catcgaccta tatctgcctc cccactaact agacagccac ctgaatctta gtctttctaa 180
   gtcccaacag taacattgcc ccaggaaatc agaccatatc agtatccctc aaagctcaag 240
   tctgtcagtg cagagccata caactaatac ccctacttat agggtaagga atggctactg 300
   ctacaggaac cagaatagct agtttgttta cttcattatc ctactaccac acactctcaa 360
   atgatttctc agacagtttg caagaaataa cgaaatctat ccttactcta caatcccaaa 420
   tagactcctt ggcagcagtg accctccaaa acggctgagg cctagacctc ctcactgcca 480
   agaaaggagg actctgcatt ttcttagggg aagagtgttt ttacactaac cagtcaggga 540
   cagtatgaga tgccactcgg agtttacagg aaaaggcttc tgaagtcaga caatgccttt 600
   caaactctat accaaactct ggagttgggc aacatggctt ctcccctttc taggtcccgt 660
   gacagccatc ttgctattat ttgcctttga gccctgtatt tttaatctcc ttttcaaatt 720
   tgtttcctct ggatcgaggc catcgagcta cagatggtct tcacaaatgg aaccccaaat 780
   gagctcaact aacaacttct actgaggacc cctggactaa cctgctgacc ctttcactgg 840
   cctgaagaat tcccctctgg aggacactac aactgcaggg ctccttcttt gcccctatcc 900
   agcaggaagt agctagagct gtcattgcct aattcctaa                        939
<210> 12
   <211> 979
   <212> ADN
   <213> Homo sapiens
<400> 12
   agtgataatg gaatacttga aagtaatccc ctcactcccc aggaactagt gctcagctgg 60
   cagaactaat agccctcact cgggtactag aatcaggaga aggaaaaagg gtaaatatat 120
   atacagactc taagtgtgct tacctagtcc tccatgccca tgcagcaata tggagagaaa 180
   gggaattcct aacttccgag ggaacaccta tcaaacatca ggaagccatt aggaaattat 240
   tattggctgt acagaaacct aaagaggtgg cagttttaca ctgccggggt catcagaaag 300
   gaaaggaaag ggaaatacaa gggagccacc aagttgatat tgaagtcaaa agagccacaa 360
   ggctggaccc tccattagaa atgcttatag gaggacccct agtatggggt aatcccctcc 420
   gggaagccaa gccccagtac tcagcaggag aaatagaata gggaacttca tgaggacata 480
   cttccctccc ctccagatgg ctagccacca ataaaggaaa aatacttttg cctgcagcta 540
   accaatagaa attacttaaa acccttcatc aaaccttcca cttaggcatt gatagcaccc 600
   atgagatggc caaattatta tttactggac caggcctttt caaaactatc aagcagatag 660
   tcagggcctg taaagtctgc caaagaaata atcccctgca ctgcaggcca tacatttcaa 720
   tccctgtatc tttaacctcc ttcttaaatt tgtctcttcc agaatcaaag ctgtaaaatt 780
   acaaatagtt cttcaaatgg agccacagat gcagtccatg actaagatcc accacagacc 840
   cctggaccag cctgctagcc catgctccaa tgttaatgac atcgaaggca ccccctcctg 900
   aggaaatctc aactgcacaa cccctactac gccccaattc agcagaaagc agttagagtg 960
   gtcatcagcc aacctcccc                                              979
<210> 13
   <211> 938
   <212> ADN
   <213> Homo sapiens
<400> 13
   tgtaggaaga actcccttca ggacaggaca atagatggtt cctcccaggt gattaaggaa 60
   aaaagacaca gtattcagta agtgataagg aaactcttgt agaagcagag ttagaaaaat 120
   tgcctaataa ttggtctgct caaatgtgtg agttgtttgc actcagccaa atcttaaagt 180
   acttacagaa tcaggaagca gccatctata ccaattctaa gttaatatgg actaaacgag 240
   gttttattag tagcaaagaa aaattaaaat cccaaactta caaggttttc aactaaagtt 300
   tgccaaaagt taacagtgta acatgtatta tcctactatc acacactctc aaaggatttc 360
   tcagacagtt tgcaagaaat aacgtaatct atccttactc tacagtccca aatagactct 420
   ttggtagcag tgactctcca aaactgccga ggtctagacc tcctcaatgc tgagaaagga 480
   gaactctgca ccttcttagg ggaagagtgc tgtttttaca ctaaccagtc agggatagta 540
   tgagatactg cctgacgttt acaggaaaag gcttctgaaa tcagacaacg cctttcaagc 600
   tcttatacca acctctggag ttgggcaaca tggcttctcc ccttgctagg tcctgtggca 660
   gccatcttgc tattacttgc cttcgggccc tgtattttta acctccttgt caaatttgtt 720
   tcctctagga tcaaggccat caagctacag atggtcttac aaatggaacc ccaaatgagc 780
   tcaactaaca acttctactg aggacacctg gactgaccca ctggcccttt cactggccta 840
   aagagttccc ttctggagga cactacaact gcagggcccc gtcttcaccc ctatccagca 900
   ggaagtagct agatcagtca ttgcccaatt cccaacag                         938
<210> 14
   <211> 540
   <212> PRT
   <213> Homo sapiens
<400> 14
   Ile Pro Met Ala Leu Pro Tyr His Ile Phe Leu Phe Thr Val Leu Leu
   1               5                   10                  15
   Pro Ser Phe Thr Leu Thr Ala Pro Pro Pro Cys Arg Cys Met Thr Ser
               20                  25                  30
   Ser Ser Pro Tyr Gln Glu Phe Leu Trp Arg Met Gln Arg Pro Gly Asn
           35                  40                  45
   Ile Asp Ala Pro Ser Tyr Arg Ser Leu Ser Lys Gly Thr Pro Thr Phe
       50                  55                  60
   Thr Ala His Thr His Met Pro Arg Asn Cys Tyr His Ser Ala Thr Leu
   65                  70                  75                  80
   Cys Met His Ala Asn Thr His Tyr Trp Thr Gly Lys Met Ile Asn Pro
                   85                  90                  95
   Ser Cys Pro Gly Gly Leu Gly Val Thr Val Cys Trp Thr Tyr Phe Thr
               100                 105                 110
   Gln Thr Gly Met Ser Asp Gly Gly Gly Val Gln Asp Gln Ala Arg Glu
           115                 120                 125
   Lys His Val Lys Glu Val Ile Ser Gln Leu Thr Arg Val His Gly Thr
       130                 135                 140
   Ser Ser Pro Tyr Lys Gly Leu Asp Leu Ser Lys Leu His Glu Thr Leu
   145                 150                 155                 160
   Arg Thr His Thr Arg Leu Val Ser Leu Phe Asn Thr Thr Leu Thr Gly
                   165                 170                 175
   Leu His Glu Val Ser Ala Gln Asn Pro Thr Asn Cys Trp Ile Cys Leu
               180                 185                 190
   Pro Leu Asn Phe Arg Pro Tyr Val Ser Ile Pro Val Pro Glu Gln Trp
           195                 200                 205
   Asn Asn Phe Ser Thr Glu Ile Asn Thr Thr Ser Val Leu Val Gly Pro
       210                 215                 220
   Leu Val Ser Asn Leu Glu Ile Thr His Thr Ser Asn Leu Thr Cys Val
   225                 230                 235                 240
   Lys Phe Ser Asn Thr Thr Tyr Thr Thr Asn Ser Gln Cys Ile Arg Trp
                   245                 250                 255
   Val Thr Pro Pro Thr Gln Ile Val Cys Leu Pro Ser Gly Ile Phe Phe
               260                 265                 270
   Val Cys Gly Thr Ser Ala Tyr Arg Cys Leu Asn Gly Ser Ser Glu Ser
           275                 280                 285
   Met Cys Phe Leu Ser Phe Leu Val Pro Pro Met Thr Ile Tyr Thr Glu
       290                 295                 300
   Gln Asp Leu Tyr Ser Tyr Val Ile Ser Lys Pro Arg Asn Lys Arg Val
   305                 310                 315                 320
   Pro Ile Leu Pro Phe Val Ile Gly Ala Gly Val Leu Gly Ala Leu Gly
                   325                 330                 335
   Thr Gly Ile Gly Gly Ile Thr Thr Ser Thr Gln Phe Tyr Tyr Lys Leu
               340                 345                 350
   Ser Gln Glu Leu Asn Gly Asp Met Glu Arg Val Ala Asp Ser Leu Val
           355                 360                 365
   Thr Leu Gln Asp Gln Leu Asn Ser Leu Ala Ala Val Val Leu Gln Asn
       370                 375                 380
   Arg Arg Ala Leu Asp Leu Leu Thr Ala Glu Arg Gly Gly Thr Cys Leu
   385                 390                 395                 400
   Phe Leu Gly Glu Glu Cys Cys Tyr Tyr Val Asn Gln Ser Gly Ile Val
                   405                 410                 415
   Thr Glu Lys Val Lys Glu Ile Arg Asp Arg Ile Gln Arg Arg Ala Glu
               420                 425                 430
   Glu Leu Arg Asn Thr Gly Pro Trp Gly Leu Leu Ser Gln Trp Met Pro
           435                 440                 445
   Trp Ile Leu Pro Phe Leu Gly Pro Leu Ala Ala Ile Ile Leu Leu Leu
       450                 455                 460
   Leu Phe Gly Pro Cys Ile Phe Asn Leu Leu Val Asn Phe Val Ser Ser
   465                 470                 475                 480
   Arg Ile Glu Ala Val Lys Leu Gln Met Glu Pro Lys Met Gln Ser Lys
                   485                 490                 495
   Thr Lys Ile Tyr Arg Arg Pro Leu Asp Arg Pro Ala Ser Pro Arg Ser
               500                 505                 510
   Asp Val Asn Asp Ile Lys Gly Thr Pro Pro Glu Glu Ile Ser Ala Ala
   515 520 525
   Gln Pro Leu Leu Arg Pro Asn Ser Ala Gly Ser Ser
   530 535 540
<210> 15
   <211> 29
   <212> ADN
   <213> Homo sapiens
<400> 15
   gatttcagta tctactagtc tgggtagat 29
<210> 16
   <211> 27
   <212> ADN
   <213> Homo sapiens
<400> 16
   ctaggaaatc cagctagtcc tgtctca 27
<210> 17
   <211> 28
   <212> ADN
   <213> Homo sapiens
<400> 17
   ccaagacagc caacttagtt gcagacat 28
<210> 18
   <211> 28
   <212> ADN
   <213> Homo sapiens
<400> 18
   ggacgctgca ttctccatag aaactctt 28
<210> 19
   <211> 29
   <212> ADN
   <213> Homo sapiens
<400> 19
   gcaatactac atacacaacc aactcccaa 29
<210> 20
   <211> 26
   <212> ADN
   <213> Homo sapiens
<400> 20
   gggggaggca tatccaacag ttagta 26
<210> 21
   <211> 30
   <212> ADN
   <213> Homo sapiens
<400> 21
   ccatctacac tgaacaagat ttatacactt 30
<210> 22
   <211> 28
   <212> ADN
   <213> Homo sapiens
<400> 22
   aatgccagta cctagtgcac ctagcact 28
<210> 23
   <211> 31
   <212> ADN
   <213> Homo sapiens
<400> 23
   cgaatacaac gtagagcaga ggagcttcga a 31
<210> 24
   <211> 28
   <212> ADN
   <213> Homo sapiens
<400> 24
   agcccaagat gcagtccaag actaagat 28
<210> 25
   <211> 27
   <212> ADN
   <213> Homo sapiens
<400> 25
   gcgtagtaga ggttgtgcag ctgagat 27
<210> 26
   <211> 27
   <212> ADN
   <213> Homo sapiens
<400> 26
   cccttaccaa gagtttctat ggagaat 27
<210> 27
   <211> 27
   <212> ADN
   <213> Homo sapiens
<400> 27
   accgctctaa ctgcttcctg ctgaatt 27
<210> 28
   <211> 32
   <212> ADN
   <213> Homo sapiens
<400> 28
   taaactacaa atggttcttc aaatggagcc ca 32
<210> 29
   <211> 32
   <212> ADN
   <213> Homo sapiens
<400> 29
   gatgcagtcc aagatgcagt ccatgactaa ga 32
<210> 30
   <211> 1740
   <212> ADN
   <213> Homo sapiens
<400> 30
   aggttggctg acaaccgctc ttaactgctt catgctgaat tggggcatag taggggtcgt 60
   gcagttgaga tttccttggg aggggtgcct tcaatgtcat caacattgga gcatgggcta 120
   gcaggccagt ccaggggtcc gcggtagatc ttagtcatgg actgcatctg gggctccatt 180
   tgaagaacca tttgtagttt tacagcttcg attctggaag agacaaacgt aacaaggagg 240
   ttaaagatac aaggattgaa atgtacggcc tgaagtgcag gggcatatga gtgtgggcgg 300
   tgcaagtggg gtttccttta gaaaaactcc gatacaatag ggcatcaata tttctaggaa 360
   gccacattct ccatagaagc tctcggtaag gggagctact ggtagtacag cagcatacag 420
   ggggtgcagt gagagtgaaa gggggtaaga gaacagtaaa aagaaaaata tgacaaggga 480
   gggccaagag gatctacgat tctagttact ttcctcacgg ttgtcgcctg aagagcaggc 540
   gcagatcctc tagaggttca caggaatagc tagcattgtc tgctggattt tcgggttcct 600
   ttggcagtat ccagggtttg gctcgagtgt gacttatcca agactccact ccagccactt 660
   aactgcggtt agggtagata aaatgactgg gtagggtcct tcccaggatg tgtgtaggga 720
   tggggaatta aaggggaagg gacttgacta ataccatgtc accagggtgg aataattcct 780
   ttccctcctc tcagggacag gttccctgta atgttttaag aactcgttga tatttggcta 840
   aggaggtgat gtctgcaact aagttggccg tctctcagtc aagcacaagg tcattggtta 900
   ggaagggctg tccatacagc atctcatatg gactaagtcc tgctttttgg ggacagtttc 960
   ggattcttag taaggctata ggcaacagag caggccatgc aaggtgggtt tcttgggtta 1020
   gcttttttag atgtcgtttg agtgtttcat tcattttctc aacttttcct gaggatcgtg 1080
   gcctccaggc acagtgtaag tgatattgta tacctaacgc ctgggatact ccctgcgtta 1140
   ctgcagcctt gaaattgggg ccattgtcac tctgtaaacc tcagggaagt ccgaatctgg 1200
   gaattatttc atgaattagt acttttatta cctcttgggc cttttctgtc ctacaaggga 1260
   aggcctccac ccaaccagtg aaagtaccca gattagtaga tactgaaatc tctgagattt 1320
   gggcatgtgg gtaaaatcta gttgctagtc ttctcctggg taatggcctg ttctttgttc 1380
   tcctgaagga gcttggcaat aaggcagggg attatttctt tggcacactt cacaggccct 1440
   gactatctgc ttgacagttt tgaaaaggcc tggtccagta aataatgatt tggccatctg 1500
   atgggtgctg tcaatgccta agtgaaaggt ctggtgaagg gttttaagta atttccattg 1560
   gttagctgca ggcaaaagta ttttttcttt ggtggctggc catcctgagg agaggaaact 1620
   atgtcctcgt gagtttcccc attccatttc ttctgctgag tactggagct tggtttccca 1680
   gaggggatta ccccatacta ggggtccttc tgtaagcatt tctaatggag agtcctgcct 1740
<210> 31
   <211> 10
   <212> PRT
   <213> Homo sapiens
<400> 31
   Phe Leu Gly Glu Glu Cys Cys Tyr Tyr Val
   1               5                   10
<210> 32
   <211> 10
   <212> PRT
   <213> Homo sapiens
<400> 32
   Leu Leu Phe Gly Pro Cys Ile Phe Asn Leu
   1               5                   10
<210> 33
   <211> 10
   <212> PRT
   <213> Homo sapiens
<400> 33
   Cys Leu Pro Leu Asn Phe Arg Pro Tyr Val
   1               5                   10
<210> 34
   <211> 10
   <212> PRT
   <213> Homo sapiens
<400> 34
   Gly Leu Leu Ser Gln Trp Met Pro Trp Ile
   1 5 10
<210> 35
   <211> 9
   <212> PRT
   <213> Homo sapiens
<400> 35
   Cys Leu Pro Ser Gly Ile Phe Phe Val
   1 5
<210> 36
   <211> 9
   <212> PRT
   <213> Homo sapiens
<400> 36
   Trp Met Pro Trp Ile Leu Pro Phe Leu
   1 5
<210> 37
   <211> 10
   <212> PRT
   <213> Homo sapiens
<400> 37
   Ile Arg Trp Val Thr Pro Pro Thr Gln Ile
   1 5 10
<210> 38
   <211> 10
   <212> PRT
   <213> Homo sapiens
<400> 38
   Leu Arg Asn Thr Gly Pro Trp Gly Leu Leu
   1 5 10
<210> 39
   <211> 10
   <212> PRT
   <213> Homo sapiens
<400> 39
   Leu Arg Thr His Thr Arg Leu Val Ser Leu
   1 5 10
<210> 40
   <211> 10
   <212> PRT
   <213> Homo sapiens
<400> 40
   Lys Arg Val Pro Ile Leu Pro Phe Val Ile
   1 5 10
<210> 41
   <211> 10
   <212> PRT
   <213> Homo sapiens
<400> 41
   Cys Arg Cys Met Thr Ser Ser Ser Pro Tyr
   1 5 10
<210> 42
   <211> 10
   <212> PRT
   <213> Homo sapiens
<400> 42
   Thr Arg Val His Gly Thr Ser Ser Pro Tyr
   1 5 10
<210> 43
   <211> 10
   <212> PRT
   <213> Homo sapiens
<400> 43
   Ala Arg Glu Lys His Val Lys Glu Val Ile
   1 5 10
<210> 44
   <211> 10
   <212> PRT
   <213> Homo sapiens
<400> 44
   Ser Arg Ile Glu Ala Val Lys Leu Gln Met
   1 5 10
<210> 45
   <211> 10
   <212> PRT
   <213> Homo sapiens
<400> 45
   Ser Gln Trp Met Pro Trp Ile Leu Pro Phe
   1 5 10
<210> 46
   <211> 9
   <212> PRT
   <213> Homo sapiens
<400> 46
   Cys Tyr Tyr Val Asn Gln Ser Gly Ile
   1 5
<210> 47
   <211> 9
   <212> PRT
   <213> Homo sapiens
<400> 47
   Phe Tyr Tyr Lys Leu Ser Gln Glu Leu
   1 5
<210> 48
   <211> 9
   <212> PRT
   <213> Homo sapiens
<400> 48
   Thr Tyr Thr Thr Asn Ser Gln Cys Ile
   1 5
<210> 49
   <211> 9
   <212> PRT
   <213> Homo sapiens
<400> 49
   Ser Phe Leu Val Pro Pro Met Thr Ile
   1 5
<210> 50
   <211> 9
   <212> PRT
   <213> Homo sapiens
<400> 50
   Tyr Tyr Val Asn Gln Ser Gly Ile Val
   1 5
<210> 51
   <211> 9
   <212> PRT
   <213> Homo sapiens
<400> 51
   Leu Phe Asn Thr Thr Leu Thr Gly Leu
   1 5
<210> 52
   <211> 9
   <212> PRT
   <213> Homo sapiens
<400> 52
   Leu Phe Gly Pro Cys Ile Phe Asn Leu
   1 5
<210> 53
   <211> 9
   <212> PRT
   <213> Homo sapiens
<400> 53
   Arg Trp Val Thr Pro Pro Thr Gln Ile
   1 5
<210> 54
   <211> 10
   <212> PRT
   <213> Homo sapiens
<400> 54
   Leu Pro Phe Leu Gly Pro Leu Ala Ala Ile
   1 5 10
<210> 55
   <211> 10
   <212> PRT
   <213> Homo sapiens
<400> 55
   Leu Pro Tyr His Ile Phe Leu Phe Thr Val
   1 5 10
<210> 56
   <211> 10
   <212> PRT
   <213> Homo sapiens
<400> 56
   Gly Ala Leu Gly Thr Gly Ile Gly Gly Ile
   1 5 10
<210> 57
   <211> 10
   <212> PRT
   <213> Homo sapiens
<400> 57
   Leu Pro Phe Val Ile Gly Ala Gly Val Leu
   1 5 10
<210> 58
   <211> 9
   <212> PRT
   <213> Homo sapiens
<400> 58
   Arg Arg Pro Leu Asp Arg Pro Ala Ser
   1 5
<210> 59
   <211> 9
   <212> PRT
   <213> Homo sapiens
<400> 59
   Phe Arg Pro Tyr Val Ser Ile Pro Val
   1 5
<210> 60
   <211> 9
   <212> PRT
   <213> Homo sapiens
<400> 60
   Arg Arg Ala Leu Asp Leu Leu Thr Ala
   1 5
<210> 61
   <211> 9
   <212> PRT
   <213> Homo sapiens
<400> 61
   Trp Arg Met Gln Arg Pro Gly Asn Ile
   1 5
<210> 62
   <211> 10
   <212> PRT
   <213> Homo sapiens
<400> 62
   Asp Arg Ile Gln Arg Arg Ala Glu Glu Leu
   1 5 10
<210> 63
   <211> 10
   <212> PRT
   <213> Homo sapiens
<400> 63
   Leu Arg Thr His Thr Arg Leu Val Ser Leu
   1 5 10
<210> 64
   <211> 10
   <212> PRT
   <213> Homo sapiens
<400> 64
   Glu Arg Val Ala Asp Ser Leu Val Thr Leu
   1 5 10
<210> 65
   <211> 10
   <212> PRT
   <213> Homo sapiens
<400> 65
   Leu Phe Gly Pro Cys Ile Phe Asn Leu Leu
   1 5 10
<210> 66
   <211> 10
   <212> PRT
   <213> Homo sapiens
<400> 66
   Gln Phe Tyr Tyr Lys Leu Ser Gln Glu Leu
   1 5 10
<210> 67
   <211> 10
   <212> PRT
   <213> Homo sapiens
<400> 67
   Gln Trp Met Pro Trp Ile Leu Pro Phe Leu
   1 5 10
<210> 68
   <211> 10
   <212> PRT
   <213> Homo sapiens
<400> 68
   Cys Tyr Tyr Val Asn Gln Ser Gly Ile Val
   1 5 10
<210> 69
   <211> 10
   <212> PRT
   <213> Homo sapiens
<400> 69
   Asn Phe Val Ser Ser Arg Ile Glu Ala Val
   1 5 10
<210> 70
   <211> 9
   <212> PRT
   <213> Homo sapiens
<400> 70
   Gly Pro Leu Val Ser Asn Leu Glu Ile
   1 5
<210> 71
   <211> 9
   <212> PRT
   <213> Homo sapiens
<400> 71
   Leu Pro Leu Asn Phe Arg Pro Tyr Val
   1 5
<210> 72
   <211> 10
   <212> PRT
   <213> Homo sapiens
<400> 72
   Leu Pro Phe Leu Gly Pro Leu Ala Ala Ile
   1 5 10
<210> 73
   <211> 10
   <212> PRT
   <213> Homo sapiens
<400> 73
   Glu Pro Lys Met Gln Ser Lys Thr Lys Ile
   1 5 10
<210> 74
   <211> 10
   <212> PRT
   <213> Homo sapiens
<400> 74
   Leu Pro Tyr His Ile Phe Leu Phe Thr Val
   1 5 10
<210> 75
   <211> 9
   <212> PRT
   <213> Homo sapiens
<400> 75
   Arg Glu Lys His Val Lys Glu Val Ile
   1 5
<210> 76
   <211> 10
   <212> PRT
   <213> Homo sapiens
<400> 76
   Lys Pro Arg Asn Lys Arg Val Pro Ile Leu
<210> 77
   <211> 9
   <212> PRT
   <213> Homo sapiens
<400> 77
   Val Val Leu Gln Asn Arg Arg Ala Leu
   1 5
<210> 78
   <211> 10
   <212> PRT
   <213> Homo sapiens
<400> 78
   Ala Val Val Leu Gln Asn Arg Arg Ala Leu
   1 5 10
<210> 79
   <211> 9
   <212> PRT
   <213> Homo sapiens
<400> 79
   Leu Pro Phe Val Ile Gly Ala Gly Val
   1 5
<210> 80
   <211> 9
   <212> PRT
   <213> Homo sapiens
<400> 80
   Asp Leu Tyr Ser Tyr Val Ile Ser Lys
   1 5
<210> 81
   <211> 10
   <212> PRT
   <213> Homo sapiens
<400> 81
<210> 82
   <211> 21
   <212> ADN
   <213> Homo sapiens
<400> 82
   cttcaaacaa caaccaggag g 21
<210> 83
   <211> 20
   <212> ADN
   <213> Homo sapiens
<400> 83
   ttggggaggt tggccgacga 20
<210> 84
   <211> 2055
   <212> DNA
   <213> Homo sapiens
<400> 84
   cccaagacag ccaacttagt tgcagacatc acctccttag ccaaatatca acaagttctt    60
   aaaacattac aaggaaccta tccctgagaa gagggaaaag aactattcca cccttgtgac   120
   atggtattag tcaagtccct tccctctaat tccccatccc tagatacatc ctgggaagga   180
   ccctacccag tcattttatc taccccaact gcggttaaag tggctggagt ggagtcttgg   240
   atacatcaca cttgagtcaa atcctggata ctgccaaagg aacctgaaaa tccaggagac   300
   aacgctagct attcctgtga acctctagag gatttgcgcc tgctcttcaa acaacaacca   360
   ggaggaaagt aactaaaatc ataaatcccc atggccctcc cttatcatat ttttctcttt   420
   actgttcttt taccctcttt cactctcact gcaccccctc catgccgctg tatgaccagt   480
   agctcccctt accaagagtt tctatggaga atgcagcgtc ccggaaatat tgatgcccca   540
   tcgtatagga gtctttctaa gggaaccccc accttcactg cccacaccca tatgccccgc   600
   aactgctatc actctgccac tctttgcatg catgcaaata ctcattattg gacaggaaaa   660
   atgattaatc ctagttgtcc tggaggactt ggagtcactg tctgttggac ttacttcacc   720
   caaactggta tgtctgatgg gggtggagtt caagatcagg caagagaaaa acatgtaaaa   780
   gaagtaatct cccaactcac ccgggtacat ggcacctcta gcccctacaa aggactagat   840
   ctctcaaaac tacatgaaac cctccgtacc catactcgcc tggtaagcct atttaatacc   900
   accctcactg ggctccatga ggtctcggcc caaaacccta ctaactgttg gatatgcctc   960
   cccctgaact tcaggccata tgtttcaatc cctgtacctg aacaatggaa caacttcagc  1020
   acagaaataa acaccacttc cgttttagta ggacctcttg tttccaatct ggaaataacc  1080
   catacctcaa acctcacctg tgtaaaattt agcaatacta catacacaac caactcccaa  1140
   tgcatcaggt gggtaactcc tcccacacaa atagtctgcc taccctcagg aatatttttt  1200
   gtctgtggta cctcagccta tcgttgtttg aatggctctt cagaatctat gtgcttcctc  1260
   tcattcttag tgccccctat gaccatctac actgaacaag atttatacag ttatgtcata  1320
   tctaagcccc gcaacaaaag agtacccatt cttccttttg ttataggagc aggagtgcta  1380
   ggtgcactag gtactggcat tggcggtatc acaacctcta ctcagttcta ctacaaacta  1440
   tctcaagaac taaatgggga catggaacgg gtcgccgact ccctggtcac cttgcaagat  1500
   caacttaact ccctagcagc agtagtcctt caaaatcgaa gagctttaga cttgctaacc   1560
   gctgaaagag ggggaacctg tttattttta ggggaagaat gctgttatta tgttaatcaa   1620
   tccggaatcg tcactgagaa agttaaagaa attcgagatc gaatacaacg tagagcagag   1680
   gagcttcgaa acactggacc ctggggcctc ctcagccaat ggatgccctg gattctcccc   1740
   ttcttaggac ctctagcagc tataatattg ctactcctct ttggaccctg tatctttaac   1800
   ctccttgtta actttgtctc ttccagaatc gaagctgtaa aactacaaat ggagcccaag   1860
   atgcagtcca agactaagat ctaccgcaga cccctggacc ggcctgctag cccacgatct   1920
   gatgttaatg acatcaaagg cacccctcct gaggaaatct cagctgcaca acctctacta   1980
   cgccccaatt cagcaggaag cagttagagc ggtcgtcggc caacctcccc aacagcactt   2040
   aggttttcct gttga                                                    2055

## Description

La présente invention est relative à une séquence nucléique et à la séquence protéique déduite, qui présente un motif rétroviral endogène humain de type *env.*

L'invention est également relative à la détection et/ou à l'utilisation de ladite séquence nucléique et de ladite séquence protéique correspondante, dans le cadre d'applications diagnostiques des neuropathologies à composante autoimmune comme la sclérose en plaques.

L'invention concerne aussi l'obtention de sondes nucléiques double brins et simple brin anti-sens, des molécules recombinantes correspondantes, et des anticorps associés.

Les rétrovirus sont des virus qui se répliquent uniquement en utilisant la voie inverse du traitement classique de l'information génétique. Ce processus, nommé transcription inverse, est médié par une ADN polymérase ARN dépendante ou transcriptase reverse, codée par le gène *pol.* Les rétrovirus codent aussi au minimum pour deux gènes additionnels. Le gène *gag* code pour les protéines du squelette, matrice, nucléocapside et capside. Le gène *env* code pour les glycoprotéines d'enveloppe. La transcription rétrovirale est régulée par des régions promotrices ou "*enhancers*", situées dans des régions hautement répétées ou LTR (*Long Terminal Repeat*) et qui sont présentes aux deux extrémités du génome rétroviral.

Lors de l'infection d'une cellule, la polymérase fait une copie ADN du génome ARN ; cette copie peut alors s'intégrer dans le génome humain. Les rétrovirus ne tuent pas les cellules qu'ils infectent, mais au contraire améliorent souvent leur rapidité de croissance. Les rétrovirus peuvent infecter des cellules germinales ou des embryons à un stade précoce ; ils peuvent dans ces conditions, intégrer la lignée germinale et être transmis par transmission mendélienne verticale, ce qui constitue la relation la plus étroite entre un hôte et son parasite (pour une revue sur les rétrovirus endogènes humains, voir notamment Patience et al., Trends in Genetics, 1997, 13, 116-120). Ces virus endogènes peuvent dégénérer au cours des générations de l'organisme hôte et perdre leurs propriétés initiales. Cependant certains d'entre eux peuvent conserver tout ou partie de leurs propriétés ou des propriétés des motifs les composant, ou encore acquérir de nouvelles propriétés fonctionnelles présentant un avantage pour l'organisme hôte, ce qui expliquerait la préservation de leur séquence.

L'existence de motifs endogènes présentant de longs cadres de lecture ouverts et/ou soumis à une forte pression de sélection peut donc être indicatrice d'une fonction biologique préservée ou acquise, qui peut correspondre à un bénéfice pour l'organisme hôte. Ces séquences rétrovirales peuvent aussi subir, au cours des générations, des modifications discrètes qui vont être à même de réveiller certaines de leurs potentialités et engendrer ou favoriser des processus pathologiques. Il est apparu récemment nécessaire de faire le bilan et d'identifier ces séquences afin de pouvoir évaluer leur impact fonctionnel.

Les séquences rétrovirales endogènes humaines ou HERVs représentent une part importante du génome humain. Ces régions rétrovirales se présentent sous plusieurs formes :
- des structures rétrovirales endogènes complètes associant des motifs *gag, pol* et *env,* flanqués de séquences nucléiques répétées, qui montrent une analogie significative avec la structure LTR-*gag*-*pol*-*env*-LTR des rétrovirus infectieux,
- des séquences rétrovirales tronquées ; par exemple, les rétro-transposons sont privés de leur domaine *env* et les rétroposons ne possèdent pas les régions *env* et LTR.

Jusqu'à présent l'étude de ces régions du génome a été négligée chez l'Homme pour deux raisons essentielles :
- l'existence d'insertions/délétions qui peuvent décaler le cadre de lecture et de mutations qui modifient la séquence. Ces modifications entraînent des altérations de la structure et par conséquent de la fonction biologique de ces motifs.
- l'absence d'associations avérées avec des pathologies humaines.

La connaissance, récente de fragments significativement représentatifs du génome humain et une orientation des recherches vers une étude structure/fonction des motifs rétroviraux endogènes, ont permis de préciser l'intérêt de ces régions. L'implication de séquences endogènes tronquées ou complètes dans des pathologies chez l'animal est documentée ; par exemple leur association avec des processus tumoraux a été clairement mise en évidence (S.K. Chattopadhyay et coll., 1982, Nature, 295, 25-31). Une recherche visant à préciser l'association ou l'influence des HERVs dans des pathologies humaines se justifie donc aujourd'hui.

Une classification des éléments HERV a été proposée (Tönjes R.R. et al., AIDS & Hum. Retrovirol., 1996, 13, S261-S267; A.M. Krieg et al., FASEB J., 1992, 6, 2537-2544). Elle est basée sur une homologie de ces séquences avec des rétrovirus isolés chez les animaux, à l'aide de sondes rétrovirales hétérologues. En effet, en général, les HERVs présentent relativement peu d'homologie avec des rétrovirus infectieux humains connus.

Les familles de classe I présentent une homologie de séquence avec les rétrovirus de mammifères de type C ; on peut citer notamment la superfamille ERI, proche du virus MuLV (*murine leukemia virus*) et du virus BaEV (*baboon endogenous virus*).

Les familles de classe II présentent une homologie de séquence avec les rétrovirus de mammifères de type B tel que le MMTV (*mouse mammary tumour virus*) ou les rétrovirus de type D tel que le SRV (*squirrel monkey retrovirus*).

D'autres familles ont également été décrites ; parmi celles-ci, on peut citer des HERVs qui présentent, de manière exceptionnelle, une homologie partielle avec HTLV-1 (RTVL-H) ou des virus de primates ; HRES-1, par exemple, présente une homologie de séquence avec des HTLVs. L'analyse du polymorphisme du LTR5' d'HRES-1 a mis en évidence une association potentielle entre HRES-1 et la sclérose en plaques (Rasmussen, H.B. et J. Clausen, Acta Neurol., Scand., 1997, 169, 32-37).

Les programmes de très grand séquençage du génome humain permettent aujourd'hui de disposer d'un nombre significatif de nouvelles séquences rétrovirales. L'usage de logiciels de traitement de données permet d'identifier et d'analyser ces gènes. Dans ce contexte une recherche systématique portant sur l'ensemble des informations disponibles à ce jour a été engagée afin d'identifier de nouvelles séquences rétrovirales endogènes humaines en fonction de certains critères d'analyse :
- présence de longs cadres de lecture ouverts conservés au cours de l'évolution de l'organisme hôte et pouvant laisser envisager une fonction biologique,
- analogie avec des séquences déjà caractérisées en dehors ou dans le domaine des rétrovirus,
- localisation dans des régions de susceptibilité pour certaines pathologies ou à proximité de gènes essentiels, par exemple dans les domaine du cancer, des régulation du système immunitaire ou dans certaines neuropathologies.

Les recherches effectuées par les Inventeurs, dans des bases de données de séquences leur ont permis d'identifier une séquence *env* d'un nouveau rétrovirus endogène dénommé HERV-7q, dont l'expression normale ou pathologique peut favoriser ou perturber un effet protecteur vis-à-vis de processus pathologiques, ou intervenir dans le déclenchement ou l'aggravation de pathologies.

La présente invention a pour objet un fragment d'acide nucléique purifié de séquence SEQ ID NO:1, correspondant au gène *env* d'un rétrovirus endogène humain, dénommé HERV-7q.

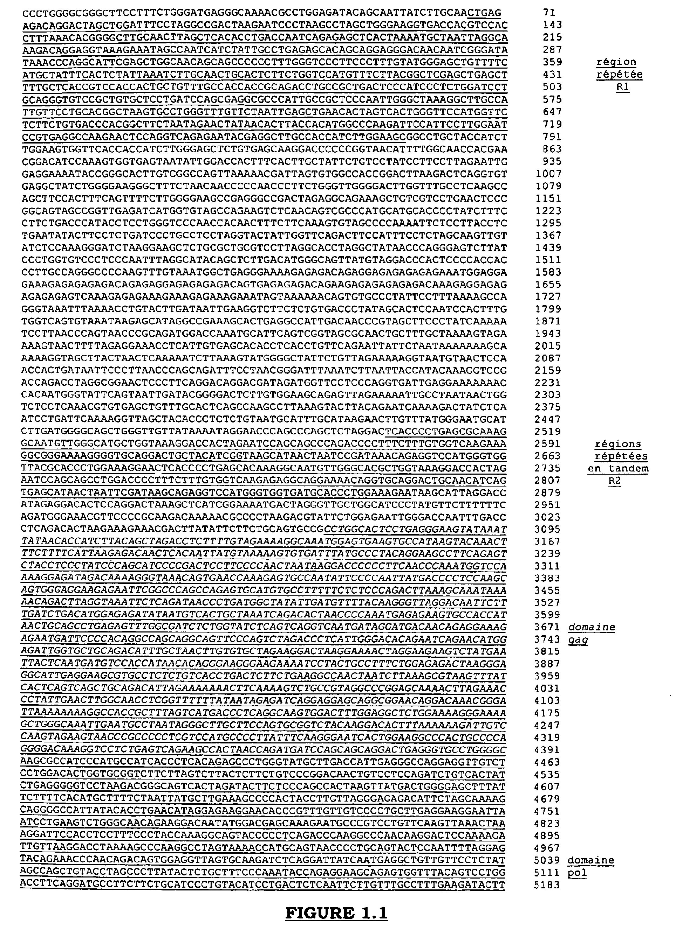

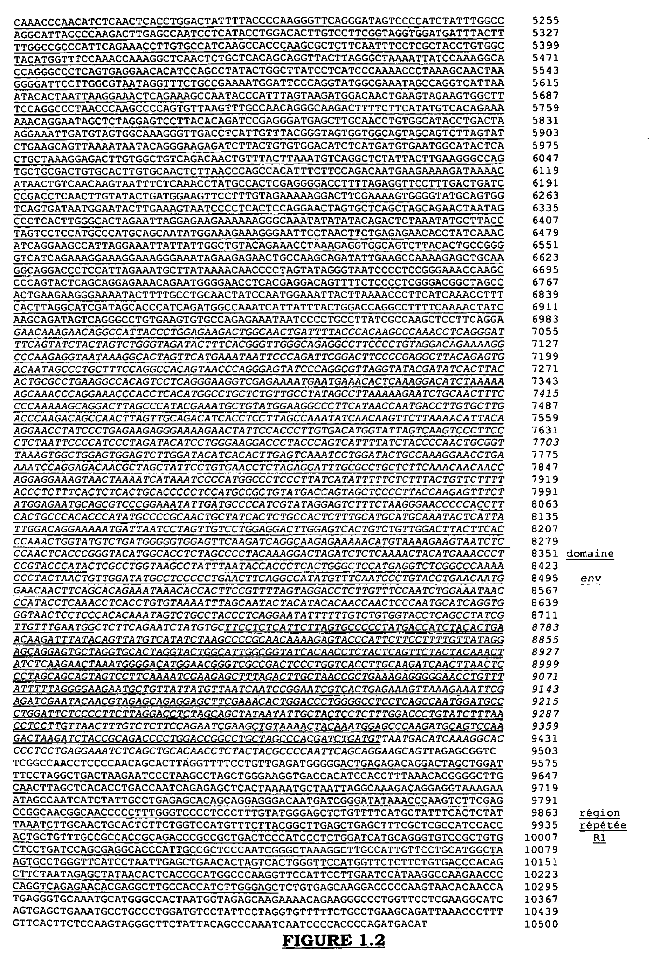

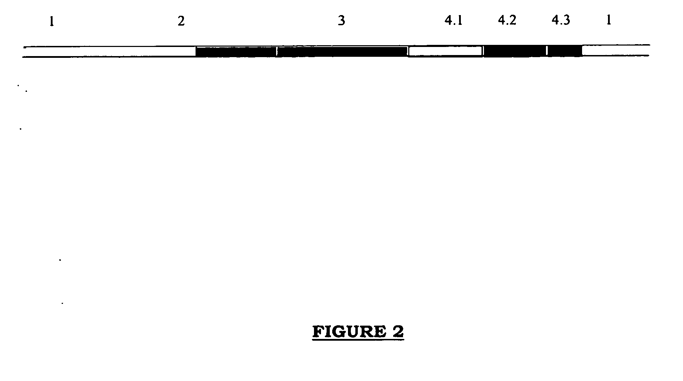

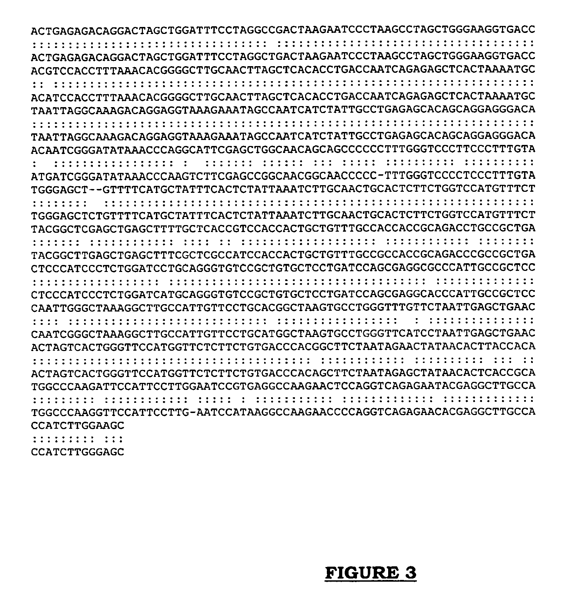

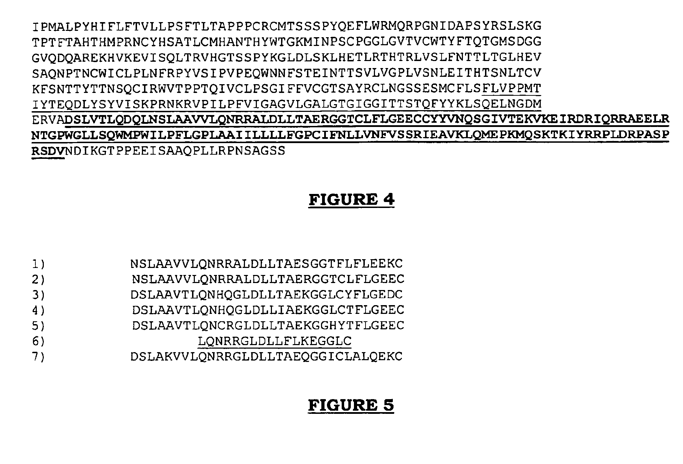

Ledit fragment est issu d'une nouvelle famille de séquences rétrovirales endogènes humaines (famille HERV-7q) qui présente une homologie de séquence avec les rétrovirus MSRV, tels que décrits dans les Demandes Internationales WO 97/06260 et WO 98/23755. La séquence HERV-7q (SEQ ID NO : 2 et figure 1) qui est située sur la chromosome 7 humain 7q21-7q22 est incluse dans le clone RG083M05 (numéro d'accès GenBank AC000064). Cette séquence (SEQ ID NO : 2) présente la structure d'un rétrovirus endogène complet et comprend :
- deux motifs nucléotidiques répétés de 711 pb (figure 3), présentant des signaux caractéristiques identifiés dans des LTRs (*Long Terminal Repeats*) *:* promoteurs de transcription de type boîtes TATAA ou CCAAT. Ces domaines répétés encadrent trois motifs déduits de type-gag, *pol* et *env* (figure 2).
- un motif de type *env* (positions 6965 nt - 9550 nt sur la séquence SEQ ID NO :2 ou sur la figure 1) qui contient un long cadre de lecture ouvert de 1617 nucléotides (positions 7879-9495 de la séquence ID NO:2 et figure 1), codant pour une protéine de séquence inédite de 538 acides aminés appelée envérine (figure 4 et SEQ ID NO:14) et fragment souligné de la figure 18. On retrouve à l'intérieur du domaine trans-membranaire de ce domaine *env,* un motif peptidique de type CKS-25/CKS-17 (figure 5), reconnu pour présenter des fonctions immunosuppressives sur les cellules lymphocytaires hôtes (M. Mitani et coll., 1987, Proc. Natl. Acad. Sci. USA, 84, 237-240). Un domaine de type doigt de zinc (*zinc-finger*) **HX₃₋₄HX₂₂₋₃₃CX₂C** (Kulkolski et coll., 1992, Mol. Cell. Biol., 12, 2331-2338), que l'on retrouve dans des domaines de type intégrase est identifié dans un autre cadre de lecture. Ce domaine *env* particulier signe la caractéristique de nouveaux motifs rétroviraux endogènes.
- le motif (positions 3065 nt - 4390 nt sur la séquence SEQ ID NO:2) de type-gag codant pour des motifs protéiques (positions 3118-4198 de la SEQ ID NO:2) a été identifié grâce à des analogies avec des domaines *gag* connus. On retrouve, par exemple, la région d'homologie majeure **QX₃EX₇R** (Benit et coll., 1997, J. Virol., 71, 5652-5657). Le motif de fixation des acides nucléiques **CX₂CX₃₋₄HX₄C,** situé en position C-terminale, est identifié dans un autre cadre de lecture (Covey et coll., 1986, Nucleic Acids Res., 14, 623-633). En amont du domaine *gag* on détecte un motif de 182 nucléotides répété deux fois (figure 1).
- le domaine *pol* présente les consensus classiques d'une région *pol* de rétrovirus au niveau des domaines protéase, transcriptase reverse et RNAse H. On retrouve dans *pol* un motif proche du consensus **LLDTGA** (Weber et coll., 1988, Science, 243, 928-931). Les motifs **D** et **AF, LPQ** et **SP,** et **YVDD** (Xiong et Eickbush, 1990, EMBO J., 9, 3353-3362), sont respectivement retrouvés dans les 3°, 4° et 5° boîtes d'homologie. Les motifs YTDGSS et TDS sont présents dans la région de la RNAse H,
- les régions *gag* et *pol* pourraient être considérées comme jointives avec un passage de la région *gag* à la région *pol* par un décalage du cadre de lecture.

La présente invention englobe également les séquences SEQ ID NO: 15-27, 28-29 et 82-83) ou leurs séquences complémentaires.

Ces différents fragments peuvent avantageusement être utilisés comme amorces ou comme sondes; ils s'hybrident spécifiquement dans des conditions de forte stringence à une séquence *env* de la famille HERV-7q.

Parmi ces fragments, on peut citer, de préférence les fragments suivants:

### Amorces et sondes spécifiques de la région env

- une amorce E1F, sens : (SEQ ID NO:15)
   5' GATTTCAGTATCTACTAGTCTGGGTAGAT 3'
- une amorce E1R, anti-sens : (SEQ ID NO:16)
   5' CTAGGAAATCCAGCTAGTCCTGTCTCA 3'
- le fragment de 2529 nt amplifié par le couple d'amorces E1F-E1R, est utilisé afin de générer les sondes aptes à hybrider les différents produits d'amplification des PCR.
- une amorce E2F, sens : (SEQ ID NO:17)
   5' CCAAGACAGCCAACTTAGTTGCAGACAT 3'
- une amorce E2R, antisens : (SEQ ID NO:18)
   5' GGACGCTGCATTCTCCATAGAAACTCTT 3'
- une amorce E3F, sens : (SEQ ID NO:19)
   5' GCAATACTACATACACAACCAACTCCCAA 3'
- une amorce E3R, anti-sens : (SEQ ID NO:20)
   5' GGGGGAGGCATATCCAACAGTTAGTA 3'
- une amorce E4F, sens : (SEQ ID NO:21)
   5' CCATCTACACTGAACAAGATTTATACACTT 3'
- une amorce E4R, anti-sens : (SEQ ID NO:22)
   5' AATGCCAGTACCTAGTGCACCTAGCACT 3'
- une amorce E5F, sens : (SEQ ID NO:23)
   5' CGAATACAACGTAGAGCAGAGGAGCTTCGAA 3'
- une amorce E6F, sens : (SEQ ID NO:24)
   5' AGCCCAAGATGCAGTCCAAGACTAAGAT 3'
- une amorce E5R : (SEQ ID NO:25)
   5' GCGTAGTAGAGGTTGTGCAGCTGAGAT 3'
- une amorce ExF : (SEQ ID NO:26)
   CCCTTACCAAGAGTTTCTATGGAGAAT
- une amorce ExR : (SEQ ID NO:27)
   ACCGCTCTAACTGCTTCCTGCTGAATT

Tous les oligonucléotides sont conçus pour pouvoir générer une amorce sens et une amorce anti-sens par un décalage de la séquence de l'amorce de référence de 1 à 7 nucléotides vers le côté 5' ou vers le côté 3': la modification de la séquence peut entraîner une modification de la taille de l'amorce de 1 à 7 nucléotides selon les cas. Les amorces choisies peuvent être optimisées selon les cas par un raccourcissement ou un allongement portant sur 1 à 9 nucléotides.

De manière préférée, l'hybridation, le clonage, le sous-clonage, l'obtention, la préparation et l'analyse des acides nucléiques, des peptides et des anticorps, le séquençage des acides nucléiques et des peptides, l'hybridation *in situ* et l'immunohistochimie sont réalisés dans les conditions décrites dans les ouvrages suivants :
- Current Protocols in Molecular Biology. Eds. F.M Ausubel, R. Brent & R.E Kingston et coll. Green Publishing associates and Wiley Interscience.
- Molecular Cloning: a laboratory manual. Eds. J. Sambrook, E.F. Fritsch & T. Maniatis. Cold Spring Harbor Laboratory Press. Cold Spring Harbor.
- The Practical Approach series. Eds. D. Rickwood & B.D. Ames. IRL Press and Oxford University Press. En particulier, antibodies 1 & II; DNA cloning I, II, III; Nucleic acid and protein sequence analysis; Nucleic acid hybridization; Nucleic acid sequencing ; Oligonucleotide synthesis; Protein purification applications; Protein purification methods; Protein sequencing; Transcription and translation; Gels electrophoresis of nucleic acids; Gels electrophoresis of proteins; Genome analysis; HPLC of macromolecules; Human genetic diseases; Microcomputing in biology; Molecular neurobiology; Mutagenicity testing; Essential molecular biology I & II.
- Proteome research: New frontiers in functional genomics. Eds M.R. Wilkins & coll.. Springer.

La séquence rétrovirale endogène humaine (SEQ ID NO:2), située sur le bras long du chromosome 7 correspond à la séquence HERV-7q ; elle présente 10,5 kb (fig. 1 et 2) et répond aux critères précédemment définis.

La recherche de domaines présentant des similitudes, tout ou partie, avec la région env de HERV-7q a abouti à l'identification de nouvelles séquences rétrovirales endogènes. Ces séquences peuvent présenter la structure d'un rétrovirus endogène complet comme la séquence rétrovirale endogène située à proximité du gène des sous-unités alpha et delta du récepteur des cellules-T, et dénommée en conséquence HERV-TcR. On trouve aussi des structures rétrovirales partielles. Ces domaines rétroviraux similaires à HERV-7q sont identifiées dans des séquences nucléiques indépendantes comme le montre leur localisation chromosomique. Des motifs nucléiques (appelés ici, HEx analogues à des domaines de type *env*) ressemblant aux domaines env de HERV-7q ont été retrouvés, à l'aide des banques de données précitées :
- HE2 : chromosome 17 (SEQ ID NO:3),
- HE3: chromosome 6 (SEQ ID NO:4),
- HE4 : chromosome X (SEQ ID NO:5),
- HE5 : chromosome X q22 (SEQ ID NO:6),
- HE6: chromosome 1 q23.3-q24.3 (SEQ ID NO:7),
- HE7 : chromosome 7 p15 (SEQ ID NO:8),
- HE8: chromosome 19 (SEQ ID NO:9),
- HE9 : chromosome X (SEQ ID NO:10),
- HE10 : chromosome X q13.1-21.1 (SEQ ID NO:11),
- HE11 : chromosome 7 q21-22 (SEQ ID NO:12),
- HE 12, dans HERV-TcR : chromosome 14 q11.2 (SEQ ID NO:13),
- HE13 (SEQ ID NO:30) : chromosome 6 q24.1-24.3

La présente invention englobe également les fragments d'au moins 14 nucléotides codant pour la partie C-terminale de l'envérine, soit à partir de l'acide aminé 291, soit à partir de l'acide aminé 321, à compter de la première méthionine.

Ces fragments comprennent en particulier une zone critique où deux insertions de 12 nucléotides ont été caractérisées :
- une première insertion a été identifiée (séquence A), chez des individus de 2 groupes (malades et témoins). Cette insertion située entre les acides aminé 487 et 488, permet d'insérer le tétrapeptide VLQM. Une analyse comparative montre que cette insertion est identifiée dans une région homologue située dans la séquence HE13, appartenant à la famille HERV-7q. L'amplification de la séquence de type HE13, pourrait indiquer qu'il existe une altération de la séquence de l'envérine de HERV-7q, ce qui favoriserait l'amplification de la séquence contenue dans HE13. Cette observation permet aussi d'utiliser cette insertion comme élément spécifique d'amplification de séquences de type HE13.

Une deuxième insertion (séquence B) a été identifiée chez un patient présentant une SEP. L'insertion de 12 nucléotides est située au niveau de l'acide aminé 495 et code pour le tétrapeptide MQSM. Il est remarquable de constater que cette insertion est aussi identifiée dans une région homologue située dans HE13.
Séquence A: TAAACTACAAATGGTTCTTCAAATGGAGCCCA (SEQ ID NO:28)
Séquence B: GATGCAGTCCAAGATGCAGTCCATGACTAAGA (SEQ ID NO:29).

Ces observations mettent en évidence des modifications de la séquence de l'envérine de type HERV-7q qui constituent la base d'une stratégie de détection par amplification spécifique d'allèles (AS-PCR), permettant de détecter ces différences dans une population et qui pourraient correspondre, soit à une mutation/délétion associée à une certaine susceptibilité, soit à un polymorphisme, soit à une mutation/délétion associée à une pathologie comme la sclérose en plaques.

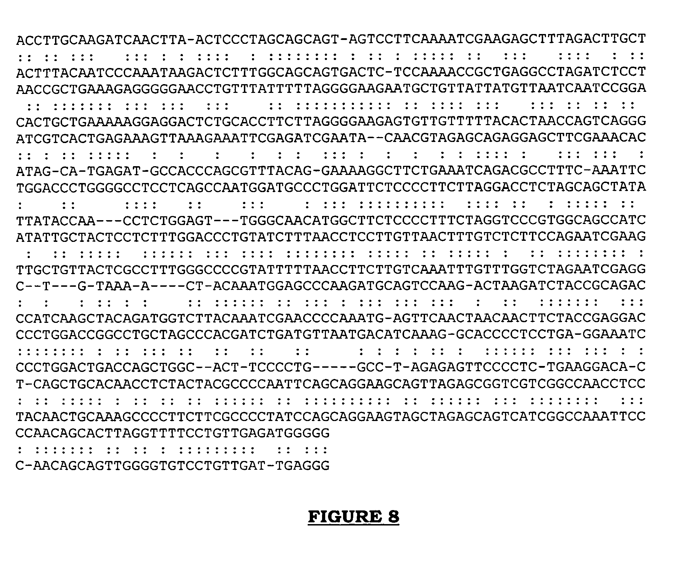

Les alignements des domaines *env* (fig. 8) explicitent les niveaux d'homologie observés entre les séquences décrites ci-dessus et les séquences homologues dans HERV-7q. Une analyse des séquences étiquettes disponibles dans les banques de données montre que des transcrits appartenant à certains des membres de cette famille, en particulier HERV-7q, s'expriment essentiellement dans des tissus d'origine foetale ou placentaire.

Des séquences polypeptidiques générées par ces transcrits peuvent donc être potentiellement produites et des fonctions ou activités biologiques peuvent être envisagées, par analogie avec des polypeptides biologiquement actifs d'origine virale ou rétrovirale ; par exemple, les motifs peptidiques de type CKS-17 (Haraguchi et al., PNAS, 1995, 92, 5568-5571) (fig. 5) ou CKS-25 (Huang S.S et Huang J.S, J. Biol. Chem. 1998, 273, 4815-4818), qui présentent des fonctions immunomodulatrices sur les cellules lymphocytaires hôtes. Les différences de séquence observées et d'éventuelles modifications normales ou pathologiques, sont en particulier, à l'origine d'une modulation de la fonction.

HERV-7q représente le paradigme de la nouvelle famille de séquences rétrovirales endogènes humaines ou de motifs rétroviraux endogènes.

HERV-7q et certaines des séquences rétrovirales endogènes appartenant à sa famille, présentent un domaine de type *pol* analogue à des séquences rétrovirales de type *pol* comme par exemple la région *pol* identifiée dans le rétrovirus MSRV associé à la sclérose en plaques et décrit par H. Perron et al. (1997, Proc. Natl. Acad. Sci. USA, 94, 7583-7588 ; Demandes Internationales PCT WO 97/06260 et WO 98/23755).

Toutefois, la séquence d'HERV-7q se distingue des séquences rétrovirales exogènes infectieuses analogues à MSRV antérieurement décrites en ce que la séquence env, selon l'invention est significativement différente en fonction de certaines caractéristiques spécifiques, par exemple le long cadre de lecture ouvert du domaine env de HERV-7q ; elle serait à même de permettre de signer une pathologie lorsqu'elles présentent des insertions, des délétions, des décalages de cadre de lecture ou des mutations.

En effet, les différences observées entre les séquences humaines *env* de type HERV-7q, qui sont isolées d'individus réputés normaux et les séquences issues de certains échantillons d'origine pathologique, ne sont pas distribuées au hasard.

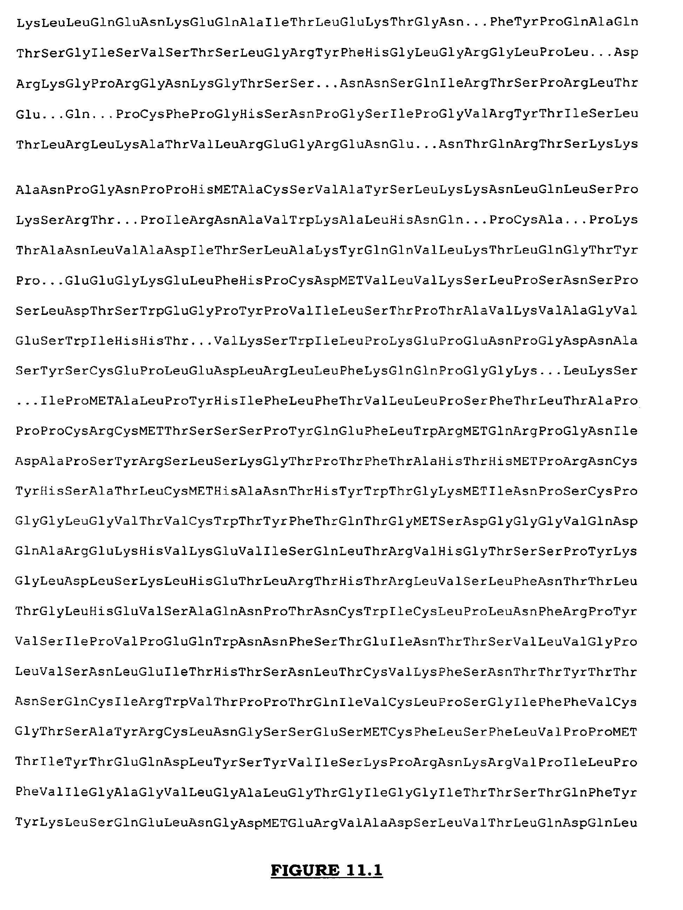

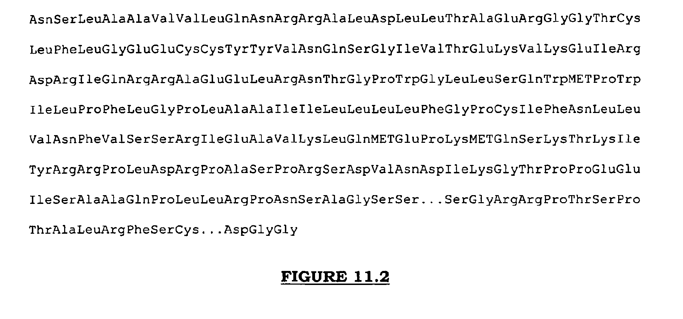

Une séquence de type env provenant de particules rétrovirales infectieuses, présente une analogie significative avec le domaine env de HERV-7q (figure 11). Ces analogies marquées entre séquences rétrovirales exogènes et endogènes pourraient être à l'origine du déclenchement ou de l'aggravation de certains processus pathologiques, en particulier de certaines maladies auto-immunes, comme la sclérose en plaques. A cet égard, on peut remarquer que certaines des séquences rétrovirales endogènes décrites dans l'invention se situent à proximité ou dans des régions réputées présenter une susceptibilité pour la sclérose en plaques : par exemple HERV-7q et la région 7q21-22 du chromosome 7, de même pour HE 12 et HG12 dans HERV-TcR et la région du gène codant pour les chaînes alpha et delta du récepteur des cellules-T, HE2 et le chromosome 17, ou HE3, HE 13 et HG3 et le chromosome 6, par exemple, les séquences HE11 et HG11, autour de la région 7q 21-22 ou encore HE4, HE5, HE6, HE9, HE10 ou HG10 sur le chromosome X. Ces séquences seraient donc à même de fournir des moyens de localisation ou d'identification des gènes de prédisposition.

On n'observe aucune homologie significative avec des séquences rétrovirales endogènes déjà décrites, par contre, on peut relever une homologie limitée, permettant d'identifier une structure générale de domaine env entre les domaines *env* de la séquence HERV-7q (SEQ ID NO :1) et de la séquence HERV-9 (figure 12). La figure 11 montre des homologies étendues entre la séquence HERV-7q avec une séquence rétrovirale exogène (N° d'accession EMBL : A60170).

Les séquences rétrovirales endogènes humaines appartenant à la famille de HERV-7q, peuvent protéger contre des agressions liées à l'environnement ou constituer un bénéfice pour l'individu. Cet effet bénéfique pourrait être une des raisons possibles de la pression de sélection exercée sur certaines de ces séquences et du caractère potentiellement fonctionnel des structures protéiques déduites identifiées : par exemple le long cadre de lecture ouvert apte à coder pour une nouvelle protéine et correspondant au domaine env de HERV-7q.

Les séquences rétrovirales endogènes humaines appartenant à la famille de HERV-7q pourraient être associées par exemple, à des pathologies en relation avec les processus liés au cancer, aux neuropathologies à composante auto-immune ou à tout autre processus pathologique en association ou non avec des virus ou rétrovirus endogènes ou exogènes. Leur action pourrait porter sur la déclaration, l'aggravation, la modification du calendrier d'apparition ou encore la protection vis à vis de la maladie.

Dans le contexte d'application à des pathologies autoimmunes (comme par exemple le lupus, le syndrome de Sjôgren, la polyarthrite rhumatoïde, la sclérose en plaques...), on peut relever des analogies significatives entre les motifs rétroviraux endogènes identifiés et des motifs retrouvés dans des structures rétrovirales caractérisées chez des patients présentant des pathologies autoimmunes comme la sclérose en plaques.

Ces motifs rétroviraux possèdent des analogies significatives avec les séquences endogènes homologues de type HERV-7q, ce qui permet d'envisager une association directe ou indirecte avec des processus pathologiques, dont la sclérose en plaques, en association ou non avec MSRV.

L'intérêt de ces séquences dépasse le cadre des maladies auto-immunes. En dehors de l'importance générale des motifs rétroviraux dans le déclenchement ou l'aggravation d'un processus tumoral, bien montré en particulier dans les modèles murins (H. Fan dans The retroviridiae, 1994, ed. J.A. Levy, Plenum, New York, p. 313-353), ces séquences pourraient se retrouver à proximité ou au sein de gènes importants et en altérer l'expression : par exemple HERV-TcR et les gènes des sous-unités alpha et delta du récepteur des cellules T impliquées dans des perturbations de la fonction immunitaire.

Enfin, il faut souligner que la région nucléique contenant HE3 est localisée au niveau du bras court du chromosome 6, en 6p21, qui est une région proposée de susceptibilité à la sclérose en plaques (The Multiple Sclerosis Genetic Group, Nature Genet., (1996), 13, 469- 472).

L'interaction entre les séquences de type HERV-7q et les séquences flanquantes et l'importance de l'établissement d'un profil d'expression incluant une ou plusieurs des séquences précitées pour établir un diagnostic différentiel d'une neuropathologie apparaît encore plus du fait que l'on observe que la séquence HE 12 est située dans une région intronique du gène codant pour les sous-unités alpha et delta des récepteurs des cellules T. Les récepteurs des cellules T sont impliqués dans les processus de régulation immunitaire et leur influence a été proposée dans le cas de maladies auto-immunes, dont la sclérose en plaques.

L'invention a également pour objet les transcrits générés à partir des séquences précitées ainsi que celles présentant éventuellement des modifications avec les séquences de référence décrites dans l'invention lorsqu'ils sont exprimés chez certains patients.

D'une manière générale des protéines rétrovirales de type HERV-7q ou apparenté, ou leurs formes tronquées ou partielles pourraient être impliquées dans des phénomènes de cytotoxicité ou de superantéginicité, comme par exemple celles issues du long cadre de lecture ouvert identifié dans le domaine *env* (figure 4).

D'une manière générale, on peut remarquer l'existence de plusieurs séquences rétrovirales endogènes de type HERV-7q (HE4, HE5, HE9, HE10), situées au niveau du chromosome X qui représente le chromosome associé au plus grandnombre de pathologies.

A cet égard, on peut relever que des motifs rétroviraux issus de régions défectives sont aptes à présenter des fonctions biologiques: par exemple, la protéine d'enveloppe p15E issue de motifs rétroviraux défectifs, possède une activité anti-inflammatoire et immunosuppressive (Snyderman et Ciancolo, 1984, Immunol. Today, 5, 240-244).

Ces structures sont vraisemblablement à même de provoquer des brèches ou d'amplifier des dérégulations dans les processus de défense immunitaire. Certains des motifs du domaine *env* peuvent être associés à une fonction particulière ou peuvent contribuer à la fonction normale ou pathologique des domaines flanquants. Des recombinaisons avec un élément d'origine exogène, rétroviral ou non, peut donner lieu à la production de motifs nucléiques ou protéiques qui pourraient soit protéger, soit déclencher, ou favoriser ou aggraver une pathologie. De même, une structure rétrovirale contenant des éléments rétroviraux endogènes selon l'invention seraient à même de provoquer un processus pathologique après passage par un cycle transitoire exogène puis réintégration dans une région sensible ou critique du génome humain.

On peut ainsi obtenir des profils d'expression (transcrits et éventuellement protéines) qui correspondent aux neuropathologies précitées.

De même, la combinaison de motifs appartenant à la famille de HERV-7q, ou d'éléments induits par des motifs appartenant à la famille de HERV-7q, avec des motifs d'origine ou induits de manière exogène seraient à même de pouvoir déclencher, ou aggraver un processus pathologique ou au contraire de favoriser une protection ou une rémission partielle ou une guérison totale et définitive.

La détection rendue possible des domaines de type HERV-7q, suggère des applications possibles à la fois au niveau prophylactique, du pronostic et du diagnostic : par exemple des approches immunologiques ou d'amplification génique permettant de comparer des individus normaux servant de référence avec des patients, seraient à même de favoriser le dépistage, d'améliorer la détection précoce de la déclaration de la maladie et/ou de suivre l'évolution d'une pathologie chez des patients pouvant présenter une susceptibilité ou ayant déclaré la maladie ou encore chez des individus considérés comme normaux, selon les critères cliniques actuels.

Les sondes nucléiques et immunologiques spécifiques, telles que définies, dans la présente invention sont à même de favoriser l'identification et la détection de motifs anormalement exprimés dans le cadre de pathologies auto-immunes, au premier rang desquelles la sclérose en plaques.

La présente invention a également pour objet un réactif de diagnostic pour la détection différentielle de séquences nucléiques endogènes humaines complètes ou partielles, présentant des motifs rétroviraux de séquence SEQ ID NO :1, caractérisé en ce qu'il est sélectionné dans le groupe constitué par les séquences SEQ ID NO:1, 15-27, 28-29 et 82-83 et les séquences nucléiques complémentaires des séquences précédentes, éventuellement marquées avec un marqueur approprié.

Les séquences des sondes nucléiques, ribonucléiques et oligonucléotidiques utilisées seront choisies dans la région env : par exemple les oligonucléotides amorces pour HERV-7q, seront choisis dans les régions situées entre, les nucléotides 6965 et 9550 de la SEQ ID NO: 2, ainsi que dans toute séquence adjacente (amont ou aval) capable de permettre une amplification spécifique (figure 1).

Parmi les marqueurs appropriés, on peut citer, les isotopes radioactifs, les enzymes, les fluorochromes, des marqueurs chimiques (biotine), les haptènes (digoxygénine) et les anticorps ou analogues de bases appropriées.

De manière préférée :
- ledit réactif est sélectionné parmi les séquences SEQ ID NO:15-27, 83-84 et est apte à être utilisé comme amorce.
- ledit réactif est sélectionné parmi les séquences suivantes :
   un fragment de 2529 nt amplifié par le couple d'amorces SEQ ID NO:15 et SEQ ID NO:16 (amorces E1F et E1R),
   des fragments d'au moins 14 nucléotides codant pour la partie C-terminale de l'envérine, soit à partir de l'acide aminé 291, soit à partir de l'acide aminé 321, à compter de la première méthionine,
et est apte à être utilisé comme sonde.

La présente invention a également pour objet un procédé de détection rapide et différentiel des séquences nucléiques rétrovirales endogènes de type *env*, de leurs variants normaux ou pathologiques, par hybridation et/ou amplification génique, réalisé à partir d'un échantillon biologique, lequel procédé est caractérisé en ce qu'il comprend :
(a) une étape dans laquelle l'on met en contact un échantillon biologique à analyser avec au moins une sonde telle que définie ci-dessus et
(b) une étape dans laquelle on détecte par tout moyen approprié, le ou les produits résultant de l'interaction séquence nucléotidique-sonde.

Conformément audit procédé, il peut comprendre :
* préalablement à l'étape (a) :
   . une étape de préparation du tissu ou du liquide biologique concerné,
   . une étape d'extraction de l'acide nucléique à détecter, et
   . au moins un cycle d'amplification génique à l'aide d'au moins une amorce telle que définie ci-dessus, et
* postérieurement à l'étape (b) :
   . une étape de comparaison des séquences nucléiques obtenues dans ledit échantillon biologique avec la séquence rétrovirale endogène humaine SEQ ID NO : 1 selon l'invention par tout moyen approprié et notamment par séquençage, Southern-blot, coupure de restriction, SSCP ou toute autre méthode permettant d'identifier une insertion ou une délétion ou encore une simple mutation entre les différentes séquences comparées.

Conformément à l'invention, la séquence rétrovirale endogène humaine selon l'invention est ainsi comparée aux séquences nucléiques présentes dans l'échantillon biologique à analyser et permett la détection de séquences homologues de patients atteints de pathologies, susceptibles de mettre en jeu une modification de leur génome.

De manière avantageuse, lesdites comparaisons géniques sont menées à partir d'ADN génomique provenant d'individus témoins et de patients.

Une amplification génique classique par PCR sera menée à l'aide d'amorces 5' -sens et 3' -antisens encadrant ou comprenant la zone à étudier (zone *env*).

Également de manière avantageuse, les séquences des sondes nucléiques, ribonucléiques et oligonucléotidiques utilisées sont choisies dans la région *env*: par exemple les oligonucléotides amorces pour HERV-7q, seront choisis dans les régions situées entre les nucléotides 6965 et 9550, ainsi que dans toute séquence adjacente (amont ou aval) capable de permettre une amplification spécifique (figure 1), comme précisé ci-dessus. De préférence ladite sonde est un fragment de 2529 nt amplifié par le couple d'amorces SEQ ID NO:15 et SEQ ID NO:16 (amorces E1F et E1R).

L'étape d'amplification génique est notamment réalisée à l'aide d'une des techniques d'amplification génique suivante : amplification par la Qβ-réplicase, PCR, LCR, ERA, CPR ou SDA.

La présente invention a également pour objet un procédé de détection des transcrits, tels que définis ci-dessus, caractérisé en ce qu'il comprend :
- le prélèvement des ARN messagers provenant d'échantillons biologiques (tissus, cellules, fluides biologiques) témoins et d'un échantillon analogue prélevé chez des patients et
- l'analyse qualitative et/ou quantitative desdits ARNm, par hybridation *in situ*, par dot-blot, Northern-blot, RNAse mapping ou RT-PCR, à l'aide d'un réactif de diagnostic tel que défini ci-dessus.

La présente invention a également pour objet une méthode de détection et/ou d'évaluation d'une sur-expression/sous-expression ou d'une modification d'au moins l'une des séquences ou fragments de séquences rétrovirales endogènes de type HERV-7q, caractérisée en ce qu'elle comprend :
- le dépôt sur un support approprié comme par exemple un filtre de nylon, une lame de verre ou leur équivalent, de l'ADNc ou son équivalent provenant de clones, de produits de PCR obtenus à partir d'ADN génomique, de produits de RT-PCR provenant de transcrits ou encore de séquences oligonucléotidiques spécifiques, lesdites séquences d'ADN étant la séquence SEQ ID NO : 1 ou des fragments de ladite séquence env d'un rétrovirus endogène dénommé HERV-7q,
- l'hybridation dudit support avec au moins une sonde marquée de manière adéquate obtenue, par exemple, par rétrotransposition d'un mélange d'ARN provenant de cellules, de tissus ou de liquides biologiques provenant de témoins réputés normaux, de membres de populations ethniques différentes, de patients atteints de pathologies souvent associées à une expression de rétrovirus, comme les maladies auto-immunes, et
- la détection des hybrides formés.

Selon un autre mode de mise en oeuvre avantageux de ladite méthode, ledit support comprend en outre toute séquence rétrovirale endogène ou exogène.

La méthode des puces à ADN (Bowtell, (1999), Nature Genet., 21, 25- 32), est utilisée pour évaluer la modification de l'expression de tout ou partie de séquences env d'un rétrovirus de type HERV-7q et des séquences flanquantes. Brièvement de l'ADN provenant de clones, de produits de PCR obtenus à partir d'ADN génomique, de produits de RT-PCR provenant de transcrits ou encore de séquences oligonucléotidiques spécifiques, sont déposées sur un support, comme par exemple un filtre de nylon, une lame de verre ou leur équivalent. Les séquences nucléiques déposées couvrent le domaine env décrit ci-dessus. Afin de détecter d'éventuels processus d'épissage alternatifs, des ADN spécifiques sont synthétisés par pas de 500- 600 nucléotides avec un chevauchement de 250- 300 nucléotides de part et d'autre.

Les épissages alternatifs déjà identifiés feront l'objet d'une synthèse spécifique. L'hybridation s'effectue à l'aide d'une sonde obtenue, par exemple, par rétrotransposition d'un mélange d'ARN provenant de cellules, de tissus ou de liquides biologiques provenant de témoins réputés normaux, de membres de populations ethniques différentes, de patients atteints de pathologies souvent associées à une expression de rétrovirus, comme les maladies auto-immunes, dont la sclérose en plaques. Dans ce cas une fraction de µg et jusqu'à quelques µg d'ARNm ou jusqu'à quelques µg ou dizaines de µg d'ARN, selon la méthode utilisée et la taille de la puce d'ADN concernée, sont suffisants pour la synthèse de la sonde nucléique. La sonde nucléique est marquée de manière adéquate, afin d'autoriser une détection ultérieure, comme par exemple par fluorescence ou par une méthode équivalente.

L'usage de sondes bi-, voire multicolores permet de préciser l'expression concertée de plusieurs gènes en parallèle, en bénéficiant de plus d'une normalisation précise. L'acquisition des résultats est effectuée automatiquement, comme par exemple par un système de balayage laser ou son équivalent.

Deux types de puces à ADN sont conçues, d'une part des puces présentant un ensemble exhaustif de séquences, et d'autre part des puces à ADN spécifiques permettant un ciblage sur une application plus spécifique.

Par exemple, une séquence critique en ce qu'elle contiendrait une différence portant sur une délétion, voire une mutation, est détectée à l'aide d'oligonucléotides spécifiques (Wang et coll., (1998), Science, 280, 1077- 1082). Le polymorphisme associé à une base ou à une mutation est détecté à l'aide de quatre oligonucléotides possédant une des quatre possibilités de séquence au niveau d'une base (A, C G ou T): pour chaque différence ponctuelle les 4 oligonucléotides sont déposés et les intensités d'hybridation sont comparées. De plus, un épissage alternatif est détecté en utilisant des ADN correspondant à un seul exon effectif ou putatif : le gène est donc analysé exon par exon.

L'étude comparative est menée entre un échantillon témoin et l'échantillon à tester, dans une perspective prophylactique, diagnostique ou thérapeutique, comme par exemple: la détection précoce d'une modification de l'expression d'une des séquences, dans une cellule, un tissu ou un organisme, l'identification d'une séquence associée à une susceptibilité ou à une pathologie quelconque, le suivi de l'évolution de la pathologie, ou encore le suivi d'un traitement et l'évaluation de son efficacité.

En dehors des applications déjà énoncées, l'intérêt de la méthode permet, d'une manière plus générale, de faire un bilan des variations constatées chez un individu, ce qui constitue en quelque sorte une carte d'identité, ce qui facilite une approche épidémiologique permettant d'établir de nouvelles corrélations entre un profil particulier observé et une pathologie, en dehors de tout *a priori* concernant cette pathologie.

La présente invention a également pour objet un kit de détection et/ou d'évaluation d'une maladie auto-immune et notamment des neuropathologies à étiologie auto-immune, caractérisé en ce qu'il comprend outre les tampons nécessaires à la mise en oeuvre des procédés tels que définis ci-dessus :
- des réactifs de diagnostic tels que définis ci-dessus.

La présente invention a également pour objet un peptide, caractérisé en ce qu'il est codé par la séquence nucléotidique SEQ ID NO:1 telle que définie ci-dessus, selon le cadre de lecture 1 (voir également figures 18-19).

Ledit peptide englobe l'envérine correspondant à une protéine de 538 aminoacides, commençant à la première méthionine de la séquence SEQ ID NO:14 et ses fragments C-terminaux, soit à partir de l'acide aminé 291, soit à partir de l'acide aminé 321, à compter de la première méthionine.

De manière avantageuse, l'analyse de la structure du domaine env de HERV-7q, appelé envérine, a permis de mettre successivement en évidence:
- un peptide signal N-terminal (région 1- 21) et deux domaines transmembranaires (région 320-340; 455-477), responsables d'interactions avec des motifs protéiques ou lipidiques membranaires,
- un motif immunomodulateur de type CKS-17(Haraguchi et coll., (1995), 92, 5568- 5571)/ CKS-25. On peut noter à cet égard, la présence d'un motif **R**al**D** à l'intérieur du peptide de type CKS-17/CKS-25 de HERV-7q et un motif **R**va**D** en position 363 qui correspondent au consensus W/RxxD, proposé pour le site actif des TGF-β (Huang et al., J. Biol. Chem., 1997, 272, 27155- 27159), de puissants facteurs associés à la croissance, à la différenciation et à la morphogenèse et qui sont associés à de nombreuses pathologies humaines, comme les processus tumoraux (Tang et coll., (1998), Nat. Med., 4, 802- 807) ou les maladies neurodégénératives (Flanders et coll., (1998), Prog. Neurobiol., 54, 71- 85). Les peptides selon l'invention contenant ces motifs peuvent avantageusement servir d'antagonistes en inhibant la fixation des TGF- P sur leurs récepteurs naturels.
- des motifs de N-glycosylation. La glycosylation des protéines d'enveloppe des rétrovirus semble être directement associée à leurs propriétés fonctionnelles, par exemple en influençant le nombre des déterminants disponibles dans les cellules-T ou en favorisant la reconnaissance des antigènes par les cellules-T. La glycosylation pourrait jouer un rôle dans la déclaration ou l'extension d'une pathologie à incidence autoimmune. Les glycosylations sont nécessaires au maintien de la conformation de certains épitopes, en particulier lors de la réalisation d'une protéine d'enveloppe recombinante à fin de mise au point d'un réactif de diagnostic et pour favoriser l'efficacité d'un éventuel vaccin. Positions 171, 210, 216, 236, 244, 283 et 411. Nombre prévu au hasard : 3.2
- des sites de prénylation. La prénylation est un mécanisme essentiel de la fixation à la membrane cellulaire et pour le ciblage de certaines protéines. Ce processus de ciblage pourrait être essentiel pour l'élaboration d'agents thérapeutiques spécifiques aptes à interférer dans la réalisation et la régulation du trafic de complexes cellulaires mettant en jeu des protéines impliquées dans les interactions, la croissance et les mouvements cellulaires. Positions 188 et 290. Nombre prévu au hasard : 1.8
- des sites de ciblage dans le réticulum endoplasmique. Ces sites permettraient d'assurer le ciblage vers le réticulum endoplasmique afin d'effectuer les modifications nécessaires pour favoriser le franchissement membranaire. Positions 353 et 431. Nombre prévu au hasard : 0.2.

Par ailleurs, les Inventeurs ont montré qu'un certain nombre de peptides issus de la protéine env de HERV-7q (envérine) présentent une affinité/demi-vie élevées pour des allèles HLA de classe I. Une analyse par CADD a permis de sélectionner des peptides candidats, dont les meilleurs scores sont indiqués dans le Tableau I:

**TABLEAU I**

| localisation | séquence | molécule HLA | score | Séquence n° |
|---|---|---|---|---|
| 399 | FLGEECCYYV | A-0201 | 7214 | SEQ ID NO:31 |
| 462 | LLFGPCIFNL | A-0201 | 1792 | SEQ ID NO:32 |
| 189 | CLPLNFRPYV | A-0201 | 1453 | SEQ ID NO:33 |
| 439 | GLLSQWMPWI | A-0201 | 488 | SEQ ID NO:34 |
| 263 | CLPSGIFFV | A-0201 | 5103 | SEQ ID NO:35 |
| 444 | WMPWILPFL | A-0201 | 897 | SEQ ID NO:36 |
| 252 | IRWVTPPTQI | B-2705 | 3000 | SEQ ID NO:37 |
| 432 | LRNTGPWGLL | B-2705 | 2000 | SEQ ID NO:38 |
| 158 | LRTHTRLVSL | B-2705 | 2000 | SEQ ID NO:39 |
| 316 | KRVPILPFVI | B-2705 | 1800 | SEQ ID NO:40 |
| 25 | CRCMTSSSPY | B-2705 | 1000 | SEQ ID NO:41 |
| 137 | TRVHGTSSPY | B-2705 | 1000 | SEQ ID NO:42 |
| 124 | AREKHVKEVI | B-2705 | 600 | SEQ ID NO:43 |
| 478 | SRIEAVKLQM | B-2705 | 600 | SEQ ID NO:44 |
| 442 | SQWMPWILPF | B-2705 | 500 | SEQ ID NO:45 |
| 405 | CYYVNQSGI | Kd | 2400 | SEQ ID NO:46 |
| 346 | FYYKLSQEL | Kd | 2400 | SEQ ID NO:47 |
| 244 | TYTTNSQCI | Kd | 2400 | SEQ ID NO:48 |
| 291 | SFLVPPMTI | Kd | 1600 | SEQ ID NO:49 |
| 406 | YYVNQSGIV | Kd | 1200 | SEQ ID NO:50 |
| 167 | LFNTTLTGL | Kd | 1152 | SEQ ID NO:51 |
| 463 | LFGPCIFNL | Kd | 960 | SEQ ID NO:52 |
| 253 | RWVTPPTQI | Kd | 480 | SEQ ID NO:53 |
| 449 | LPFLGPLAAI | B-5102 | 2200 | SEQ ID NO:54 |
| 3 | LPYHIFLFTV | B-5102 | 1210 | SEQ ID NO:55 |
| 331 | GALGTGIGGI | B-5102 | 798 | SEQ ID NO:56 |
| 321 | LPFVIGAGVL | B-5102 | 550 | SEQ ID NO:57 |
| 499 | RRPLDRPAS | B-2705 | 600 | SEQ ID NO:58 |
| 194 | FRPYVSIPV | B-2705 | 600 | SEQ ID NO:59 |
| 383 | RRALDLLTA | B-2705 | 600 | SEQ ID NO:60 |
| 39 | WRMQRPGNI | B-2705 | 600 | SEQ ID NO:61 |
| 423 | DRIQRRAEEL | B14 | 1800 | SEQ ID NO:62 |
| 158 | LRTHTRLVSL | B14 | 600 | SEQ ID NO:63 |
| 359 | ERVADSLVTL | B14 | 540 | SEQ ID NO:64 |
| 463 | LFGPCIFNLL | Kd | 1658 | SEQ ID NO:65 |
| 345 | QFYYKLSQEL | Kd | 1152 | SEQ ID NO:66 |
| 443 | QWMPWILPFL | Kd | 691 | SEQ ID NO:67 |
| 405 | CYYVNQSGIV | Kd | 500 | SEQ ID NO:68474 |
| | NFVSSRIEAV | Kd | 480 | SEQ ID NO:69 |
| 221 | GPLVSNLEI | B-5102 | 1320 | SEQ ID NO:70 |
| 190 | LPLNFRPYV | B-5102 | 726 | SEQ ID NO:71 |
| 449 | LPFLGPLAAI | B-5101 | 1144 | SEQ ID NO:72 |
| 488 | EPKMQSKTKI | B-5101 | 968 | SEQ ID NO:73 |
| 3 | LPYHIFLFTV | B-5101 | 629 | SEQ ID NO:74 |
| 125 | REKHVKEVI | Kk | 1000 | SEQ ID NO:75 |
| 312 | KPRNKRVPIL | B7 | 800 | SEQ ID NO:76 |
| 378 | VVLQNRRAL | Db | 792 | SEQ ID NO:77 |
| 377 | AVVLQNRRAL | Db | 660 | SEQ ID NO:78 |
| 321 | LPFVIGAGV | B-5101 | 629 | SEQ ID NO:79304 |
| | DLYSYVISK | A3 | 540 | SEQ ID NO:80 |
| 301 | TEQDLYSYVI | Kk | 500 | SEQ ID NO:81 |

Ce Tableau I indique une estimation de la demi-vie de dissociation d'un peptide de l'envérine avec un allèle du système HLA de classe I (les tables de coefficients de Parker: J. Immunol, (1994), 152, 163- 175). La localisation indique la position du premier acide aminé des peptides testés dans la séquence de l'envérine. Le code à une lettre est utilisé pour la séquence des acides aminés. Les scores autour de 500 ou supérieurs à 500 ont été retenus. A titre de comparaison, une analyse a été effectuée sur une concaténation de peptides (polypeptide de 4968 acides aminés) réputés pour fixer les molécules du complexe majeur d'histocompatibilité de classe 1 (Rammensee, Immunogenetics, (1995), 41, 178- 228): les dix meilleurs scores enregistrés pour des nonapeptides et le type HLA, A_0201 sont respectivement de 4984, 4047, 2406, 1267, 800, 705, 607, 591, 591 et 577.

Il ressort de ce Tableau I que certaines molécules du complexe majeur d'histocompatibilitè de type I sont aptes à fixer des peptides issus de l'envérine, ainsi assimilés à des peptides d'origine virale ou tumorale, au niveau du réticulum endoplasmique. Les complexes formés au niveau du réticulum endoplasmique sont alors transportés à la surface cellulaire, ce qui entraîne la destruction de la cellule cible par les lymphocytes-T cytotoxiques. Les peptides identifiés comportent généralement 8 à 10 acides aminés. Des études ont montré que certains allèles du système HLA de classe I sont ainsi associées à certaines pathologies, en particulier à caractère auto-immun, comme HLA-B27 avec la spondylarthrite ankylo-sante ou HLA-B51 avec la maladie de Behçet.

Un peptide apte à fixer une molécule particulière de classe I est par conséquent apte à fonctionner comme un épitope de cellule-T.

Les produits protéiques générés par les séquences rétrovirales endogènes ou produits parallèlement peuvent avantageusement être caractérisés par des micro-méthodes d'analyse et de quantification des peptides et des protéines: HPLC/FPLC ou équivalent, électrophorèse capillaire ou équivalent, techniques de microséquençages (méthode d'Edman ou équivalent, spectrométrie de masse...).

L'invention a également pour objet des anticorps dirigés spécifiquement contre un peptidesélectionné dans le groupe constitué par les peptides situés des positions 293 à 540 et 323 à 540 de la séquence SEQ ID NO : 14 et leur utilisation soit pour la mise en oeuvre d'une méthode de détection *in vitro,* notamment différentielle de la présence d'une telle séquence chez un individu.

Lesdits anticorps sont avantageusement des anticorps polyclonaux ou monoclonaux obtenus par une réaction immunologique d'un organisme humain, mammifères, oiseaux ou autres espèces vis-à-vis des protéines, telles que définies ci-dessus.

La présente invention a pour objet un procédé de dépistage immunologique différentiel de séquences rétrovirales endogènes humaines *env* de la famille HERV-7q normales ou pathologiques, caractérisé en ce qu'il comprend la mise en contact d'un échantillon biologique avec un anticorps selon l'invention, la lecture du résultat étant révélée par un moyen approprié, notamment EIA, ELISA, RIA, fluorescence.

A titre d'illustration, une telle méthode de diagnostic *in vitro* selon l'invention comprend la mise en contact d'un échantillon biologique prélevé chez un patient, avec des anticorps selon l'invention et la détection à l'aide de tout procédé approprié, notamment à l'aide d'anti-immunoglobulines marquée, des complexes immunologiques formés entre les protéines produites normalement ou pathologiquement et les anticorps.

Des anticorps monoclonaux ou polyclonaux, produits à partir d'antigènes correspondants à des peptides de synthèse, de polypeptide ou protéines recombinants, permettent de suivre l'expression des peptides ou protéines produits normalement ou pathologiquement. L'analyse est de préférence effectuée par ELISA ou équivalent, Western-blot ou équivalent, ou par immunohistochimie.

Les peptides ou protéines, issus de la séquence rétrovirale endogène *env*, sont recherchés et identifiés.

La présente invention a également pour objet un procédé d'identification et de détection de motifs rétroviraux endogènes, anormalement exprimés dans le cadre de pathologies associées au cancer, ou de neuropathologies à composantes auto-immunes, au premier rang desquelles la sclérose en plaques, caractérisé en ce qu'il comprend l'analyse comparée des séquences extraites d'un échantillon biologique avec la séquence SEQ ID NO : 1 selon l'invention.

La présente invention a également pour objet l'application des séquences nucléiques ou des séquences protéiques selon l'invention au diagnostic, au pronostic, à l'évaluation de la susceptibilité génétique, à toute neuropathologie humaine induite, innée ou acquise à composantes autoimmunes, comme la sclérose en plaques, les syndromes associés et les maladies neurodégénératives où intervient tout ou partie de la séquence nucléique selon l'invention.

La présente invention a, en outre, pour objet un vecteur recombinant d'expression, caractérisé en ce qu'il comprend une séquence nucléique.sélectionnée dans le groupe constitué par la séquence SEQ ID NO : 1, une séquence de 2529 nucléotides située des positions 7053 à 9581 de la séquence SEQ ID NO : 2 et une séquence codant le peptide des positions 293 à 540 ou 323 à 540 de la séquence SEQ ID NO : 14.

La présente invention a également pour objet des animaux transgéniques non-humains, caractérisés en ce qu'ils comprennent tout ou partie de la séquence SEQ ID NO:1.

Le Tableau II ci-après établit les correspondances entre les numéros des séquences telles qu'elles apparaissent dans la liste de séquences et le nom des différentes séquences.

**TABLEAU II**

| **SEQ ID NO :** | **DÉSIGNATION** |
|---|---|
| 1 | Acide nucléique : 7 env |
| 2 | Acide nucléique : HERV-7q |
| 3 | Acide nucléique : HE2 |
| 4 | Acide nucléique : HE3 |
| 5 | Acide nucléique : HE4 |
| 6 | Acide nucléique : HE5 |
| 7 | Acide nucléique : HE6 |
| 8 | Acide nucléique : HE7 |
| 9 | Acide nucléique : HE8 |
| 10 | Acide nucléique : HE9 |
| 11 | Acide nucléique : HE10 |
| 12 | Acide nucléique : HE11 |
| 13 | Acide nucléique : HE12 |
| 14 | Protéine : envérine |
| 15 | Acide nucléique : E1F (amorce) |
| 16 | Acide nucléique : E1R (amorce) |
| 17 | Acide nucléique : E2F (amorce) |
| 18 | Acide nucléique : E2R (amorce) |
| 19 | Acide nucléique : E3F (amorce) |
| 20 | Acide nucléique : E3R (amorce) |
| 21 | Acide nucléique : E4F (amorce) |
| 22 | Acide nucléique : E4R (amorce) |
| 23 | Acide nucléique : E5F (amorce) |
| 24 | Acide nucléique : E6F (amorce) |
| 25 | Acide nucléique : E5R (amorce) |
| 26 | Acide nucléique : ExF (amorce) |
| 27 | Acide nucléique : ExR (amorce) |
| 28 | Acide nucléique : Séquence A (séquence d'insertion) |
| 29 | Acide nucléique : Séquence B (séquence d'insertion) |
| 30 | Acide nucléique : HE13 |
| 31 | Peptide Tableau I |
| 32 | Peptide Tableau I |
| 33 | Peptide Tableau I |
| 34 | Peptide Tableau I |
| 35 | Peptide Tableau I |
| 36 | Peptide Tableau I |
| 37 | Peptide Tableau I |
| 38 | Peptide Tableau I |
| 39 | Peptide Tableau I |
| 40 | Peptide Tableau I |
| 41 | Peptide Tableau I |
| 42 | Peptide Tableau I |
| 43 | Peptide Tableau I |
| 44 | Peptide Tableau I |
| 45 | Peptide Tableau I |
| 46 | Peptide Tableau I |
| 47 | Peptide Tableau I |
| 48 | Peptide Tableau I |
| 49 | Peptide Tableau I |
| 50 | Peptide Tableau I |
| 51 | Peptide Tableau I |
| 52 | Peptide Tableau I |
| 53 | Peptide Tableau I |
| 54 | Peptide Tableau I |
| 55 | Peptide Tableau I |
| 56 | Peptide Tableau I |
| 57 | Peptide Tableau I |
| 58 | Peptide Tableau I |
| 59 | Peptide Tableau I |
| 60 | Peptide Tableau I |
| 61 | Peptide Tableau I |
| 62 | Peptide Tableau I |
| 63 | Peptide Tableau I |
| 64 | Peptide Tableau I |
| 65 | Peptide Tableau I |
| 66 | Peptide Tableau I |
| 67 | Peptide Tableau I |
| 68 | Peptide Tableau I |
| 69 | Peptide Tableau I |

| **SEQ ID NO:** | **DÉSIGNATION** |
|---|---|
| 70 | Peptide Tableau I |
| 71 | Peptide Tableau I |
| 72 | Peptide Tableau I |
| 73 | Peptide Tableau I |
| 74 | Peptide Tableau I |
| 75 | Peptide Tableau I |
| 76 | Peptide Tableau I |
| 77 | Peptide Tableau I |
| 78 | Peptide Tableau I |
| 79 | Peptide Tableau I |
| 80 | Peptide Tableau I |
| 81 | Peptide Tableau I |
| 82 | Acide nucléique : F645 (amorce) |
| 83 | Acide nucléique : PS5D (amorce) |
| 84 | Acide nucléique + protéine env déduite : HERV-7q |

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- Figure 1. Séquence nucléique humaine HERV-7q, dont l'analyse et le traitement permettent de caractériser une nouvelle structure rétrovirale endogène. Les régions nucléiques répétées de type R1 et R2 et les domaines *gag, pol* et *env* sont soulignés. Les domaine de type *gag* et *env* sont en italiques. La région homologue à une partie 3' non-codante de Rab7 est doublement soulignée.
- Figure 2. Cartographie de la région rétrovirale endogène humaine HERV-7q. La partie haute de la figure correspond à une région anonyme du génome humain située sur le bras long du chromosome 7. On peut identifier les domaines répétés (1), *gag* (2), *pol* (3) et *env* (4) de HERV-7q. La région *env* C-terminale (4.3) se prolonge en amont en un long cadre de lecture ouvert (4.2). Le domaine 4.1, correspond à la région N-terminale du domaine *env.*
- Figure 3. Comparaison des séquences nucléiques répétées situées aux bornes de HERV-7q. Les régions nucléiques répétées 5'(haut) et 3'(bas), sont comparées et les bases identiques sont indiquées par deux points.
- Figure 4. Séquence déduite présentant un cadre de lecture ouvert, dans le domaine de type-env de HERV-7q selon la règle du plus long cadre de lecture ouvert.
- Figure 5. Séquences autour du domaine CKS-17 identifiées dans différents domaines env déduits de la famille de HERV-7q et comparaison avec des motifs CKS-17 de référence.
   1) HE2 - 2) HERV-7q - 3) N° d'accès à GenBank: M85205 - 4) HE7 - 5) HE9 - 6) CKS-17: le motif peptidique doué de propriétés immunomodulatrices est souligné - 7) gp20 de rétrovirus de type-D (SRV-Pc).

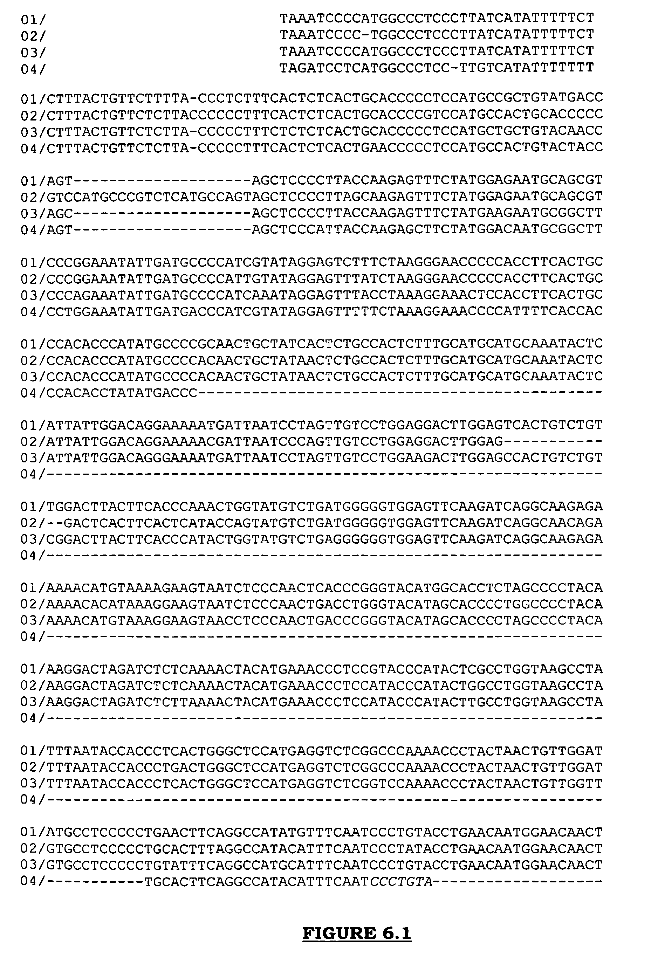

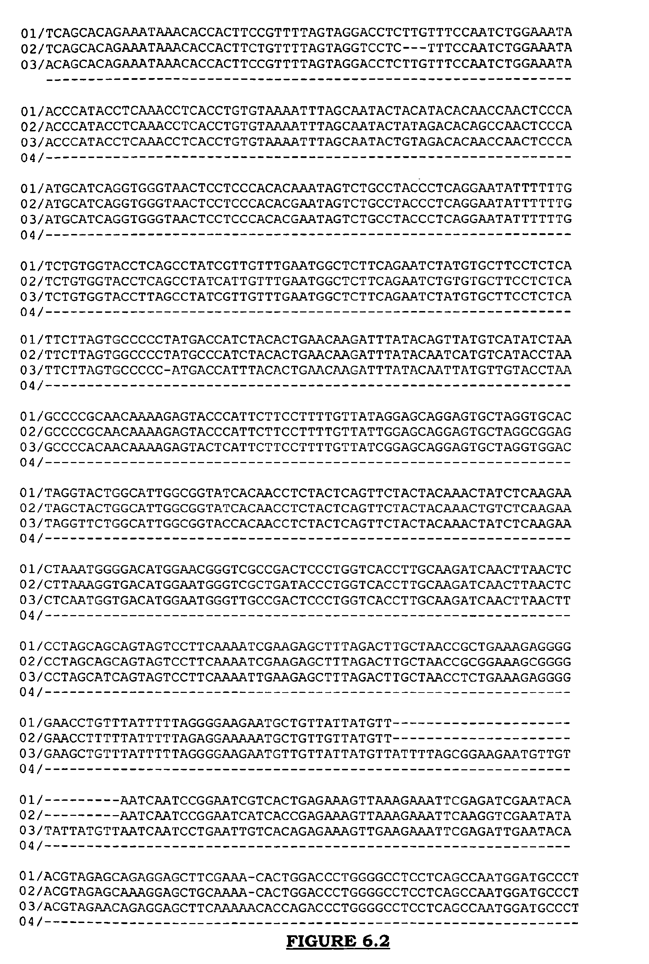

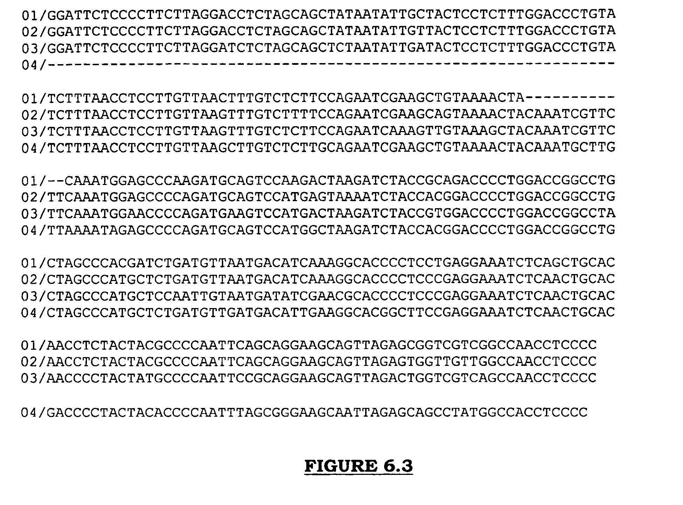

- Figure 6. Exemple d'alignements nucléiques du domaine de type *env* de HERV-7q avec des domaines de type env similaires présents dans des séquences rétrovirales endogènes humaines de la même famille. Les codons non sens sont soulignés : 1) HERV-7q - 2) HE2 - 03) HE3 - 04) HE4.

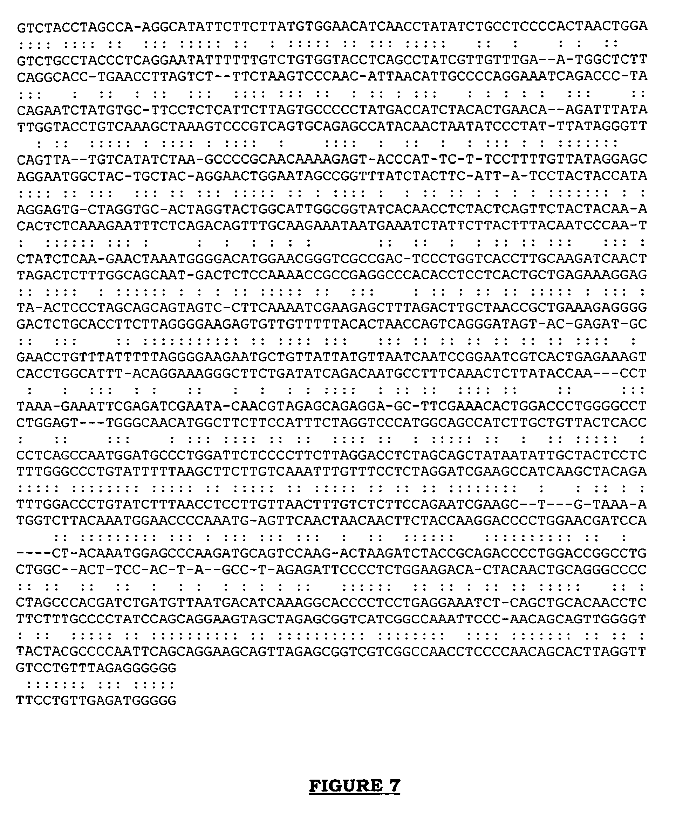

- Figure 7. Alignement d'un motif env (haut) appartenant à un rétrovirus infectieux (N° d'accession EMBL : A60170) avec le motif env (bas) identifié dans HERV-7q. Les nucléotides homologues sont spécifiés par deux points et les délétions par un tiret.
- Figure 8. Comparaison entre le domaine env de HERV-7q (haut) et le domaine env de HERV-9 (bas). L'homologie de 66 % se limite à la région 3' du domaine env de HERV-7q et HERV-9, respectivement entre les nucléotides 8976 nt et 9500 nt de HERV-7q et les nucléotides 2898 nt et 3465 nt de HERV-9 (N° d'accession à GenBank : X57147). De nombreuses insertions/délétions sont aussi observées.

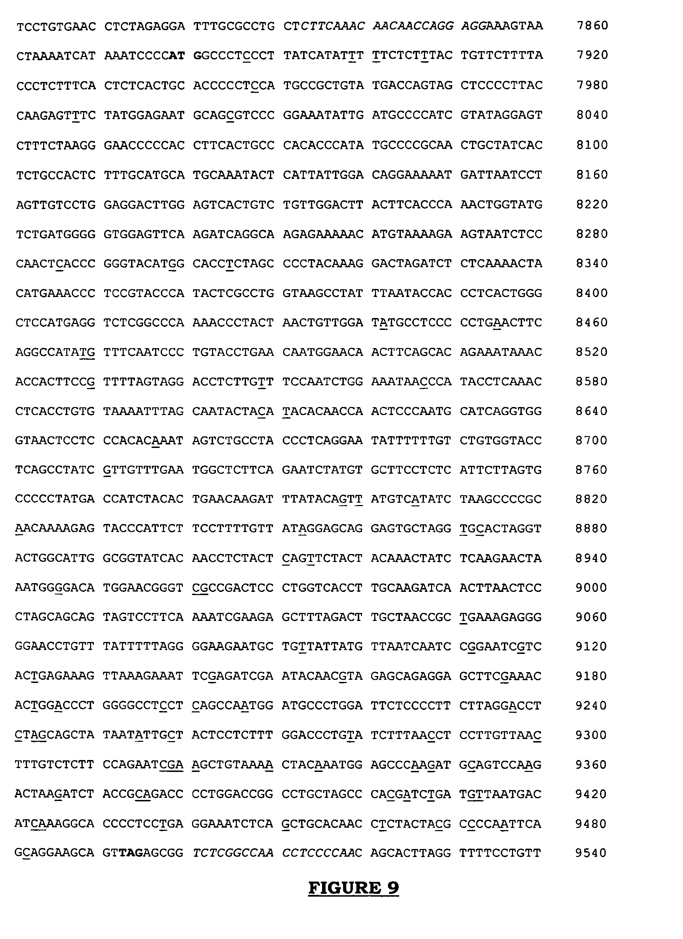

- Figure 9. Mise en évidence de modifications sur la séquence nucléotidique (ID NO:3), chez des patients atteints de SEP. Les bases modifiées, chez au moins un patient, sont soulignées. Les amorces utilisées sont en italiques
   (séquences SEQ ID NO:82 et 83). L'ATG d'initiation et le codon non-sens sont en gras.

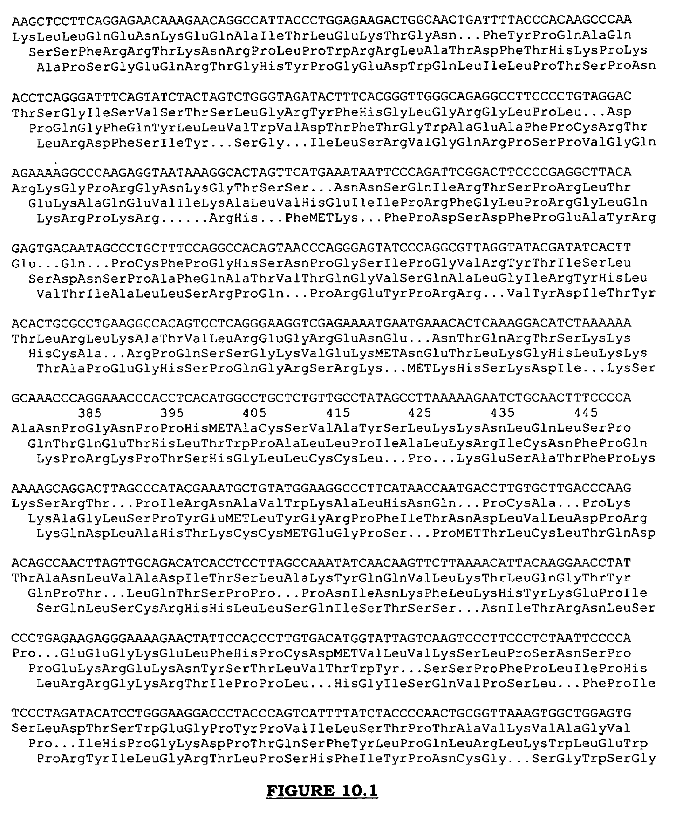

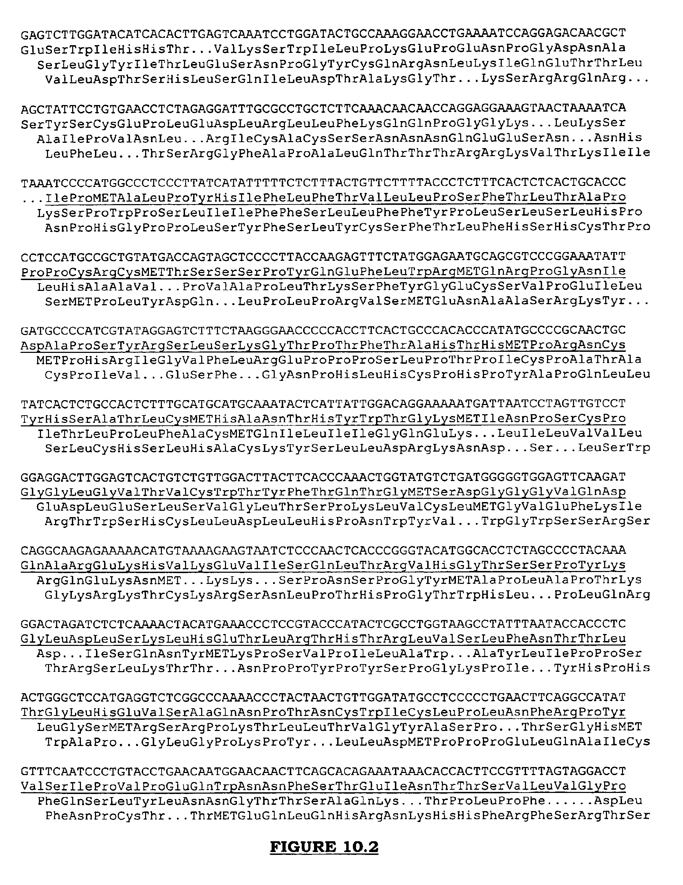

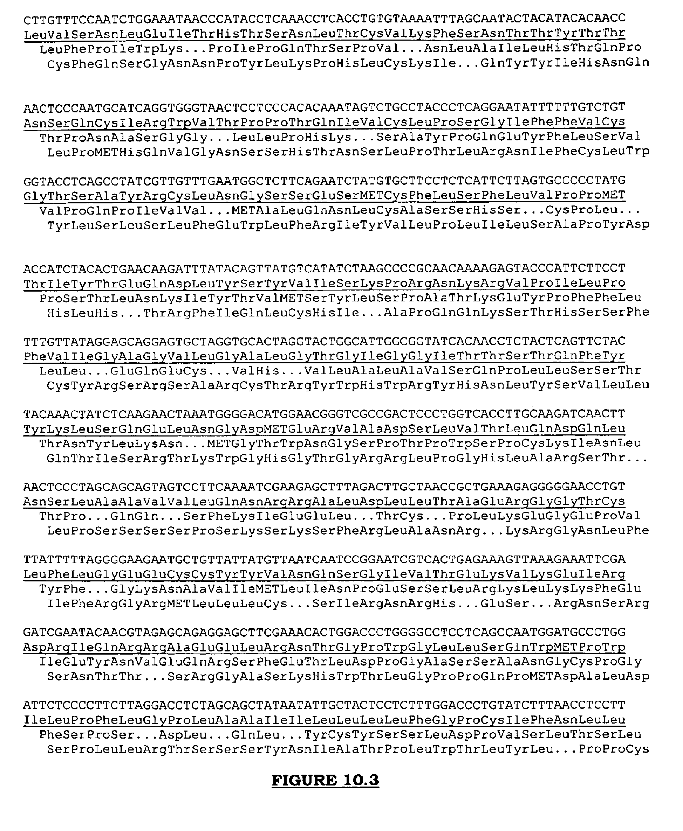

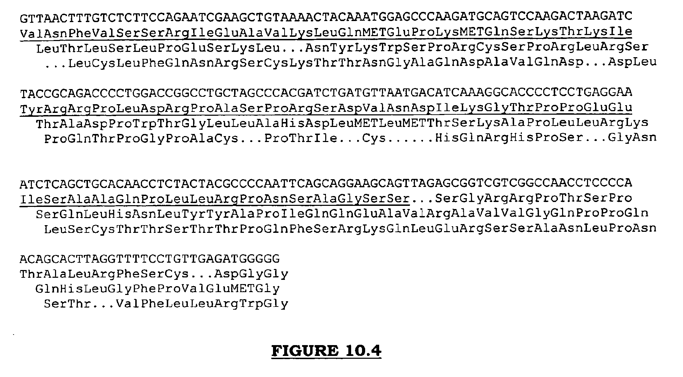

- Figure 10. Partie codante *env* de la séquence HERV-7q (séquence ID NO:2), avec 3 cadres de lecture.
- Figure 11. Présentation de la protéine env selon son cadre de lecture.

### EXEMPLE 1 : Détection, par amplification génique, d'une séquence nucléique appartenant à un domaine de type env selon l'invention, dans un échantillon d'ADN génomique d'origine humaine ou de mammifères.

L'amplification génique s'effectue à partir d'ADN génomique isolé à partir du sang. Un traitement anticoagulant est effectué avec 1 ml d'une solution de citrate (pour un litre : 4,8 g de d'acide citrique, 13,2 g de citrate de sodium, 14,7 g de glucose) pour 6 ml de sang frais. Après centrifugation de 20 ml de sang pendant 15 mn à 13.0000 g, le surnageant est éliminé et la fraction enrichie en globules blancs est transférée dans un nouveau tube, puis recentrifugée dans les mêmes conditions que précédemment. La fraction enrichie en globules blancs est resuspendue dans un tampon d'extraction (10 nm Tris-HCl, 0,1 M EDTA, 20 µg/ml de RNAse pancréatique traitée afin d'éliminer les DNAses, 0,5 % SDS, pH 8,0), puis incubée pendant 1 heure à 37°C. La protéinase K est ajoutée à une concentration finale de 100 µg/ml. La suspension des cellules lysées est incubée à 50°C durant 3 heures sous agitation périodique, puis traitée par un volume égal de phénol équilibré par du Tris-HCl 0,5 M, pH 8,0. L'émulsion formée est placée sur une roue pendant une heure, puis centrifugée à 5000 g pendant 15 mn à température ambiante. La solution aqueuse est traitée déprotéinisé par une triple extraction phénolique afin d'obtenir un niveau de purification correspondant à un rapport final d'absorbance A260/A280 supérieur à 1,75. La fraction aqueuse est précipitée par 0,2 vol. d'acétate de sodium 10 M et 2 vol. d'éthanol. L'ADN est alors soit prélevé avec l'extrémité d'une pipette pasteur recourbée, soit centrifugé à 5000 g pendant 5 mn à température ambiante. L'ADN ou le culot d'ADN est lavé deux fois par de l'éthanol à 70 %, puis repris dans 1 ml de TE pH 8,0 afin d'être élué sous agitation douce pendant 12 à 24 heures.

Des oligonucléotides spécifiques des séquences endogènes décrites selon l'invention sont choisis pour amplifier la région *env* des régions rétrovirales endogènes décrites selon l'invention. L'ADN génomique étudié provient de patients présentant des pathologies comme la sclérose en plaques et d'individus réputés sains.

Les ADN polymérases thermostables utilisées ont été choisies pour leur grande fidélité lors du processus d'amplification, comme la Ventᵣ ADN polymérase (Biolabs) ou équivalent, et sont utilisées selon les conditions préconisées par le fournisseur.

La stratégie d'amplification utilise selon les cas une simple PCR, ou une PCR nichée ou semi-nichée.

Oligonucléotides utilisés pour amplifier la région env de HERV-7q :
- amorce E1F, sens (SEQ ID NO:15),
- amorce E1R, anti-sens (SEQ ID NO:16).

Le fragment de 2529 nt amplifié par le couple d'amorces E1F-E1R, est utilisé afin de générer les sondes aptes à hybrider les différents produits d'amplification des PCR.
- amorce E2F, sens (SEQ ID NO:17),
- amorce E2R, antisens (SEQ ID NO:18),
- amorce E3F, sens (SEQ ID NO:19),
- amorce E3R, anti-sens (SEQ ID NO:20),
- amorce E4F, sens (SEQ ID NO:21),
- amorce E4R, anti-sens (SEQ ID NO:22),
- amorce E5F, sens (SEQ ID NO:23),
- amorce E6F, sens(SEQ ID NO:24)
- amorce E5R(SEQ ID NO:25).
- amorce ExF (SEQ ID NO:26)
- amorce ExR (SEQ ID NO:27)

La PCR est réalisée à partir de 50 à 200 ng d'ADN génomique. Les conditions de PCR sont celles préconisées par le fournisseur. Les conditions cycliques d'amplification sont réalisées dans 50 µl : une dénaturation de 94°C pendant 1 min., une hybridation de 70°C pendant 1 min., et une élongation à 72 °C pendant 1 à 2 min., selon les fragments amplifiés. Après 35 cycles, une réaction terminale est menée à 72°C pendant 10 min. Le séquençage automatique des échantillons amplifiés est réalisé à l'aide d'un séquenceur Applied Biosystems de type ABI 377 ou autre modèle comparable, selon les protocoles fournis par le constructeur.

Dans le cas d'une PCR nichée ou semi-nichée, les mêmes conditions expérimentales sont utilisées, à la seule différence que l'échantillon d'ADN génomique est remplacé par 5 à 10 µl du produit d'amplification issu de la première PCR.

Deux amplifications indépendantes sont réalisées à partir du même échantillon. Une réaction de contrôle est réalisée en remplaçant l'échantillon d'ADN par de l'eau afin de détecter d'éventuels contaminants.

### EXEMPLE 2 : Détection par amplification génique d'une séquence nucléique selon l'invention dans un échantillon biologique d'ADN génomique prélevé chez des patients présentant une pathologie candidate déclarée ou la suspicion de cette pathologie.

Le protocole d'amplification est le même que dans l'exemple 1, mis à part l'origine de l'échantillon qui provient de patients présentant une pathologie candidate. Un échantillon d'ADN génomique réputé normal est systématiquement intégré dans l'ensemble des échantillons pathologiques amplifiés puis analysés.

Les produits de PCR sont séparés sur un gel d'agarose à 1,5 %, puis transférés en présence de soude 0,4 N sur une membrane de nylon chargé. Une hybridation est réalisée avec une sonde spécifique correspondant au fragment de PCR amplifié par le couple E1F-E1R. La sonde est marquée par incorporation de dUTP-digoxygénine selon le protocole du fournisseur (Boehringer Mannheim). L'hybridation est effectuée dans un tampon d'hybridation (5XSSC, 50 % formamide, 0,1 % lauroyl-sarcosine, 0,02 % SDS, 2 % de réactif de blocage Boehringer) pendant une nuit à 42°C. Le Southern est lavé 2 fois 5 min. à température ambiante dans une solution de 2XSSC, 0,1% SDS. Puis un lavage à haute stringence est effectué à deux reprises pendant 15 min. à 55°C dans une solution 0.1XSSC, 0,1 % SDS. L'hybridation est révélée selon le protocole du fournisseur (Boehringer Mannheim), en présence d'un substrat chimioluminescent de la phosphatase alcaline, de type CSPD ou CDP-STAR. Le filtre est révélé après une exposition
de 15min. à 60 min.

Une analyse par SSCP ("*single strand conformation polymorphism*") permet de détecter des modifications discrètes de la séquence des fragments amplifiés par PCR. La PCR est menée en présence de dCTP marqués au P³². L'échantillon a analyser est dénaturé à 95°C pendant 10 min., en présence de tampon de charge, puis immédiatement chargé sur un gel de polyacrylamide à 10%, contenant 7.5% de glycérol. La migration s'effectue à 4°C à 8-10 W. Le gel est séché puis auto-radiographié.

Les fragments de PCR susceptibles de présenter une altération de leur séquence nucléotidique sont séquencés selon l'exemple 1.

Une hybridation à l'aide d'un oligonucléotide spécifique (17 mers à 20 mers) correspondant à la région nucléotidique modifiée permet d'identifier les échantillons présentant une modification identique (méthode ASO). Brièvement le southem est hybridé avec un oligonucléotide marqué distalement soit au P³², soit en présence de digoxygénine (selon le protocole de Boehringer Mannheim) puis lavé dans des conditions stringentes à 65%C dans une solution 6XSSC, 0.05% pyrophosphate de sodium.

Par exemple, un séquençage nucléotidique automatique a été réalisé sur six fragments de PCR, provenant de 5 patients atteints de SEP et un témoin réputé normal, et qui ont été amplifiés à partir des amorces F645: CTTCAAACAACAACCAGGAGG (SEQ ID NO:82) (située à 26 nucléotides en amont de la méthionine d'initiation de l'envérine) et PS5D: TTGGGGAGGTTGGCCGACGA (SEQ ID NO:83) (située à 6 nucléotides en aval du codon non-sens de l'envérine. Des modifications de la séquence de l'envérine ont été observés sur l'ADN de certains des patients (figure 17).

### EXEMPLE 3 : Détection d'une protéine selon l'invention dans un échantillon biologique.

### - Préparation d'une fraction protéique purifiée de liquide céphalo-rachidien de patients atteints de SEP

Après un traitement à 56°C pendant 30 min, et élimination des immunoglobulines sur une colonne de protéine G HiTrap (Pharmacia), l'équivalent de 10 ml de LCR est déposé sur une colonne de DEAE Sepharose CL-6B (Pharmacia). L'élution est réalisée en Tris-HCl 20 mM pH 8,8, et un gradient de 0 à 0,4 M de NaCl, puis la fraction est dialysée 2 fois contre du tampon phosphate-NaCl (PBS). Après concentration sur Ultrafree-MC (Millipore), la fraction est déposée sur une colonne de Superose 12 (FPLC Pharmacia) et éluée en présence de PBS. Après séparation par électrophorèse en gel de polyacrylamide-SDS, et électo-transfert sur une membrane d'Immobilon-P (Millipore), les bandes protéiques sont soumises à une hydrolyse trypsique ménagée.

### - Analyse de la fraction protéique par spectrométrie de masse

Les peptides digérés en présence de trypsine, sont analysés par la méthode de MALDI-TOF, qui permet l'analyse de peptides présents en mélange. (COTTRELL J.S., Pept. Res., 1997, 7, 115-124). Les peptides caractérisés en fonction de leur masse sont comparés aux protéines et aux protéines associées selon l'invention.

### EXEMPLE 4 : Détection d'anticorps spécifiques anti-domaine env de HERV-7q.

L'identification d'un long cadre de lecture ouvert au sein de la séquence env de HERV-7q, a permis de déterminer une séquence protéique déduite (figures 18-19) d'une région dudit gène.

La séquence de la protéine déduites des figures 18-19 (cadre de lecture 1) est positionnée comme suit par rapport à la figure 1 ou à la séquence ID NO:2: début de la séquence codante : position 7879, fin de la séquence codante 1^{er} codon non-sens (position 9495).

Différents peptides correspondant à tout ou partie de la SEQ ID NO: 14 ont été synthétisés par génie génétique afin de tester leur spécificité antigénique vis-à-vis de séra ou de tissus de patients atteints de SEP, par exemple. Brièvement, tout ou partie de la région env de HERV-7q est sous clonée dans les vecteurs pQE30, 31 et 32. Les vecteurs pQE30, 31 et 32 contiennent en 5' du multi-site de clonage les séquences consensuelles pour la transcription (le promoteur fort du bactériophage T5, 2 opérateurs de l'opéron lactose), la traduction (un site d'accrochage ribosomal synthétique). De même, pQE30, 31 et 32 possèdent en 3', le terminateur de transcription du phage 1 ainsi qu'un codon "Stop" pour la traduction. L'expression de la protéine s'effectue après transformation dans *E. coli* M15. Le plasmide pQE30, 31 et 32 possèdent en amont du site de polyclonage la séquence codante pour une suite de 6 histidines présentant une affinité pour les ions nickel. Cet enchaînement permet la purification de la protéine chimérique exprimée, par adsorption sur une résine constituée d'un ligand chélatant, l'acide nitrilotriacétique (NTA), chargé de 4 ions nickel (résine NI-NTA, Qiagen).

La transformation s'effectue par électroporation ou traitement au chlorure de calcium. Par exemple, une colonie d'*E. coli* M15 est incubée dans 100 ml de milieu LB contenant 250 µg de kanamycine, sous agitation à 37°C jusqu'à l'obtention d'une DO⁶⁰⁰ de 0,5. Après une centrifugation de 5 minutes à 2000g à 4°C, le culot bactérien est repris dans 30 ml de solution TFB1 (100 mM de chlorure de rubinium, 50 mM de chlorure de manganèse, 30 mM d'acétate de potassium, 10 mM CaC12, 15% glycérol, pH 5.8), à 4°C pendant 90 minutes. Après une centrifugation de 5 minutes à 2000g à 4°C, le culot bactérien est repris dans 4 ml de solution TFB2 (10 mM de chlorure de rubidium, 10 mM de MOPS, 75 mM CaCl2, 15% de glycérol , pH 8). Les cellules peuvent être gardées à -70°C par aliquot de 500 ml. 20 µl de la ligation et 125 µl de cellules compétentes sont mélangés et placés dans la glace 20 minutes. Après un choc thermique de 42°C pendant 90 secondes, les cellules sont agitées 90 minutes à 37°C dans 500 ml de milieu Psi-broth (milieu LB complémenté par 4 mM de MgSO₄, 10mM de chlorure de potassium). Les cellules transformées sont étalées sur des boîtes LB-agar complémentées par 25 µg/ml de kanamycine, et 100µg/ml d'ampicilline, et les boîtes sont incubées une nuit à 37°C.

Les clones potentiellement recombinants sont repiqués de manière ordonnée sur un filtre de nylon déposé sur une boîte LB-agar complémentée par 25 µg/ml de kanamycine et 100 µg/ml d'ampicilline. Après une nuit à 37°C, les clones recombinants sont repérés par hybridation de l'ADN plasmidique avec la sonde nucléotidique amplifiée par PCR avec le couple d'amorces selon SEQ ID NO: 15 et SEQ ID NO: 16.

Une colonie indépendante, contenant l'insert, est inoculée à 20 ml de milieu LB complémentée par 25 µg/ml de kanamycine et 100 µg/ml d'ampicilline. Après une nuit à 37°C sous agitation, 500 ml de même milieu sont incubés au 1/50° par cette préculture jusqu'à l'obtention d'une D0⁶⁰⁰ de 0,8, puis 1 à 2 mM final d'IPTG est ajouté. Après 5 heures, les cellules sont centrifugées 20 minutes à 4000 g.

Une partie du culot cellulaire est repris dans 5 ml de tampon de sonication (50 mM de phosphate de sodium pH 7,8, 300 mM NaCl) puis placé dans la glace. Après une rapide sonication, les cellules sont centrifugées 20 minutes à 10000 g. Une partie du culot cellulaire est repris dans 10 ml d'une solution 30 mM Tris/HCl-20% sucrose pH8. Les cellules sont incubées 5 à 10 minutes sous agitation, après adjonction de 1 mM EDTA. Après une centrifugation de 10 minutes à 8000 g à 4°C, le culot est repris dans 10 ml de 5 mM de MgSO4 glacé. Après 10 minutes dans la glace sous agitation, les cellules sont centrifugées 10 minutes à 8000 g à 4°C.

Le culot est repris par 5 ml/g dans du tampon A (6 M GuHCl (chlorhydrate de guanidine), 0,1M phosphate de sodium, 0,01M Tris/HCl, pH 8), 1 heure à température ambiante. Le lysat est centrifugé 15 minutes à 10000 g à 4°C, et le surnageant est complémenté par 8 ml de résine Ni-NTA, prééquilibrée dans du tampon A. Après 45 minutes à température ambiante, la résine est coulée dans une colonne, lavée par 10 fois le volume de la colonne par du tampon A puis par 5 fois le volume da la colonne par du tampon B (8 M urée, 0,1 M phosphate de sodium, 0,01 M Tris/HCl, pH 8). La colonne est lavé par du tampon C (8 M urée, 0,1M phosphate de sodium, 0,01 M Tris/HCl, pH 6,3) jusqu'à ce que l'A280 soit inférieur à 0,01. La protéine recombinante est éluée par 10 à 20 ml de tampon D (8 M urée, 0,1 M phosphate de sodium, 0,01 M Tris/HCl, pH 5,9) puis par 10 à 20 ml de tampon E (8 M urée, 0,1 M phosphate de sodium, 0,01 M Tris/HCl, pH 4,5), puis par 20 ml de tampon F (6 M HCl, 0,2 M acide acétique). Après une analyse en SDS-PAGE, la ou les fractions purifiées contenant la protéine chimérique ont permis l'obtention d'anticorps chez le lapin. Les anticorps obtenus sont testés par Western-blot après révélation par un anticorps secondaire couplé à la phosphatase alcaline.

Des anticorps sont obtenus de la même manière, à partir de peptides synthétisés chimiquement selon la technique de Merrifield (G. Barany and B. Merrifield, 1980, dans The peptides, 2, 1-284, E. Gross et J. Meienhofer, Academic Press, New York).

Les anticorps spécifiques obtenus sont utilisés à fin de détection de l'expression sérique ou tissulaire de tout ou partie des séquences rétrovirales endogènes selon l'invention, dans les cas normaux et pathologiques.

Les protéines d'origine sérique ou tissulaire, sont séparées sur gel d'acrylamide-SDS puis transférées sur un filtre de nitrocellulose à l'aide d'un appareil Novablot 2117-2250 (LKB). Le transfert est effectué sur une feuille de Hybond C-extra (Amersham) en utilisant un tampon CAPS 100 mM pH 11, méthanol, eau (V/V/V: 1/1/8) contenant 1 mM de CaCl₂. Après un transfert de 1 heure à 0,8 mA/cm², la feuille est saturée une heure à température ambiante dans du PBS-0,5 % gélatine. La feuille est mise en présence de l'anticorps spécifique à la concentration de 1/1000 dans du PBS-0,25 % gélatine. Au bout de 2 heures, le filtre est lavé 3 fois 15 minutes dans du PBS-0,1 % de Tween-20, puis le filtre est incubé 30 minutes en présence d'un anticorps secondaire couplé à la phosphatase alcaline (Promega), dilué au 1/7500 dans du PBS-0,25% gélatine. Après trois lavages dans du PBS-0,1 % de Tween-20, le filtre est équilibré dans un tampon (100 mM de Tris-HCl pH 9,5, 100 mM de NaCl, 5 mM de MgCl₂). La révélation est effectuée en présence de 45 µl de NBT à 75 mg/ml et 35 µl de BCIP à 50 mg/ml, pour 10 ml de tampon de phosphatase alcaline.

### EXEMPLE 5 : Obtention de sondes ribonucléiques codant pour les séquences env de HERV-7q.

Les fragments de PCR obtenus sont sous clonés dans le plasmide PGEM 4Z (Promega) qui possède de par et d'autre de son site de polyclonage, les séquences promotrices pour les ARN polymérase SP6 et T7.

La méthode de compétence utilisée est l'électroporation. Le plasmide et le fragment de PCR sont hybridés dans un rapport de 50 ng de vecteur (coupé à Sma I) pour 100 ng de fragment de PCR (rendu à bout franc par traitement par le fragment de Klenow de l'ADN polymérase). L'incubation a lieu une nuit à 22°C, dans le tampon de ligation (66 mM Tris-HCl pH 7,5, 5 mM MgCl2, 1 mM dithioerythritol, 1 mM ATP) en présence de 1u. de T4 ADN ligase puis est arrêtée par dénaturation 10 minutes à 65°C. Parallèlement, la souche d*'E*. *Coli* JM 105 est ensemencée une nuit à 37°C dans du milieu LB. Cette préculture est diluée au 1/500 et placée à 37°C jusqu'à une DO⁶⁰⁰ égale à 1. Pour la suite du mode opératoire les cellules seront toujours conservées au froid. Après une centrifugation de 5 minutes à 3500 g à 4°C, le culot cellulaire est resuspendu dans 1/4 vol. d'eau glacée ultra-pure. Cette étape est répétée 5 à 6 fois. Puis le culot est resuspendu dans 1/4000 vol. d'eau; 10 % de glycérol stérile sont ajoutés permettant la conservation des cellules électrocompétentes, par aliquots de 10 µl à 20°C. A 50 µl de cellules électrocompétentes est ajouté 1 µl de la ligation ; le tout est soumis à une décharge électrique de 12,5 kV/cm, appliquée pendant 5,8 ms. Les cellules sont rapidement remises en suspension dans le milieu SOC, incubées 1 heure à 37°C, puis étalées, en présence de 2% X-Gal dans du diméthylformamide, et 10 mM d'IPTG, sur une boîte de gélose LB-agar supplémentée en ampicilline (100 µg/ml). Après une nuit à 37°C, les clones blancs potentiellement recombinants, sont repiqués de manière ordonnée sur une boîte LB/ampicilline et parallèlement sur un filtre de nylon déposé sur une boîte LB/ampicilline. Ces deux boîtes sont incubées une nuit à 37°C. Les clones recombinants sont alors repérés par hybridation avec une sonde nucléique amplifiée par PCR avec le couple d'amorces selon SEQ ID NO: 15 et SEQ ID NO: 16 et marquée à la digoxygénine.

Les clones recombinants sont cultivés dans 50 ml de milieu LB/ampicilline (100 µg/ml) en agitation pendant une nuit à 37°C. Après une centrifugation à 3500 g pendant 15 minutes à 4°C, le culot bactérien est repris dans 4ml de tampon P1 (50 mM Tris-HCl, 10mM EDTA, 400 µg/ml RNase A, pH 8) et 4ml de tampon P2 (200 mM NaOH, 1% SDS). Le mélange est incubé à température ambiante pendant 5 minutes. Après adjonction de 4ml de tampon P3 (2,55 M d'acétate de potassium, pH 4,8) le mélange est centrifugé à 12000 g pendant 30 minutes à 4°C. Le surnageant est appliqué sur une colonne Qiagen-type 100, prééquilibrée avec 2 ml de tampon QBT (750 mM NaCl, 50 mM MOPS, 15% éthanol, pH 7), la colonne est lavée avec 2 fois 4ml de tampon QC (1M NaCl, 50 mM MOPS, 15 % éthanol, pH 7) et l'ADN est élué avec 2ml de tampon QF (1,2 M NaCl, 50mM MOPS, 15 % éthanol, pH 8). L'ADN est précipité avec 0,8 vol. d'isopropanol, et centrifugé à 12000 g à 4°C pendant 30 minutes. Le culot est lavé avec de l'éthanol à 70 % glacé, puis l'ADN plasmidique est repris par 2 fois 150 µl de tampon TE.

Les sondes ribonucléiques sont utilisées comme sondes spécifiques, en particulier pour la détection des transcrits exprimés par les séquences rétrovirales endogènes selon l'invention.

### EXEMPLE 6 : Construction d'une souris transgénique contenant tout ou partie du gène de l'envérine.

Une souris transgénique contenant tout ou partie de la séquence du gène env d'HERV-7q (SEQ ID NO:1) est construite afin d'identifier les séquences responsables de la spécificité tissulaire, et pour évaluer le rôle de tout ou partie des motifs peptidiques de l'envérine. La technique de micro-injection utilisée se réfère à la technique classique (Hogan et coll., (1994), Manipulating the mouse embryo, Cold Spring Harbor, Cold Spring Harbor Laboratory Press) ou à ses équivalents. Des formes identiques à l'envérine humaine normale ou des formes mutées, délétées, présentant des insertions ou tronquées sont testées afin de déterminer les motifs critiques tant sur le plan normal que pathologique, et plus particulièrement au cours du développement foetal et lors des processus tumoraux.

### Bibliographie :

- Benit L. et al., 1997. Cloning of a new murine endogenous retrovirus MuERV-L, with strong similarity of the human HERV-L element and with a gag coding sequence closely related to the Fv1 restriction gene. J. Virol. 71, 5652-5657.
- Coffin J.M. 1985. Endogenous retrovirus. In: " RNA tumor viruses " (Weiss R.A., Varmus H.E., Teich N.M., and Coffin J.M. eds), Cold Spring Harbor Laboratory Press, Cold Spring Harbor, New York.
- Conrad B., Weissmahr R.N., Boni J., Arcari R., Schupbach J., and Mach B. 1997. A human endogenous retroviral superantigen as candidate autoimmunogene in type 1 diabetes. Cell 90, 303-313.
- Covey S.N. 1986. Amino acid sequence homology in gag region of reverse transcribing elements and the coat protein gene of cauliflower mosaic virus. Nucleic Acids Res. 14, 623-633.
- Hertig C., Coupar B.E., Gould A.R., and Boyle D.B. 1997. Field and vaccine strains of fowlpox virus carry integrated sequences from the avian retrovirus, reticuloendotheliosis virus. Virology 235, 367-376.
- Hohenadl C., Leib-Mösch C., Hehlemann R., and Erfle Y. 1996. Biological significance of human endogenous retroviral sequences. J. Acqui. Imm. Def. Synd. Hum. Retrovir. 13, S268-S273.
- Kulkoski J.K., Jones S., Katz R.A., Mack J.P.G., and Skalka A.M. 1992. Residues critical for retroviral integrative recombination in a region that is highly conserved among retroviral/retrotransposon integrases and bacterial insertion sequence transposases. Mol. Cell. Biol. 12, 2331-2338.
- La Mantia G. et al, N.A.R., 1991, 19, 7, 1513-1520
- Patience C., Wilkinson D.A., and Weiss R.A. 1997. Our retroviral heritage. Trends Genet. 13, 116-120.
- Pearson W.R. 1994. Using the FASTA program to search protein and DNA sequence databases. Methods Mol. Biol. 24, 307-331.
- Perron H., Garson J.A., Bedin F., Beseme F., Paranhos-Baccala G., Komurian-Pradel F., Mallet F., Tuke P.W., Voisset C., Blond J.L., Lalande B., Seigneurin J.M., Mandrand B. and the Collaborative Research Group on Multiple Sclerosis. 1997. Molecular identification of a novel retrovirus repeatedly isolated from patients with multiple sclerosis. Proc. Natl. Acad. Sci. USA 94, 7583-7588.
- Tönjes R.R. et al., J. AIDS and Hum. Retrovirol, 1996, 13, S261-S267
- Vitelli R., Chiarillo M., Lattero D., Bruni C.B., and Bucci C. 1996. Molecular cloning and expression analysis of the human Rab7 GTP-ase complementary deoxyribonucleic acid. Biochem. Biophys. Res. Commun. 229, 887-890.
- Weber L.T., Miller M., Jaskolski M., Leis J., Skalka M., and Wlodawer A. 1989. Molecular modeling of the HIV-1 protease and its substrate binding site. Science 243, 928-931.
- Wilkinson D., Mager D.L., and Leong J.A.C. 1994. Endogenous human retroviruses. In: "The Retroviridae " (Levy, J.A. ed), Plenum Press New York, , Vol. 3, 465-535.
- Xiong Y., and Eickbush, T. 1990. Origin and evolution of retroelements based upon their reverse transcriptase sequences. EMBO J. 9, 3353-3362.

## Revendications

1. Polynucléotide isolé de séquence SEQ ID NO : 1, correspondant au gène env d'un rétrovirus endogène humain dénommé HERV-7q.

2. Polyribonucléotide susceptible d'être obtenu par transcription de la séquence telle que définie à la revendication 1.

3. Sonde ou amorce pour la détection de séquences rétrovirales endogènes humaines de la famille HERV-7q, **caractérisée en ce qu'**elle est spécifique du gène *env* et qu'elle est sélectionnée dans le groupe constitué par :
a) la sonde de 2529 nucléotides située des positions 7053 à 9581 de la séquence SEQ ID NO : 2, susceptible d'être amplifiée par le couple d'amorces SEQ ID NO:15 et SEQ ID NO:16,
b) les sondes ou amorces présentant les séquences SEQ ID NO : 15 à 27, 28-29, 82-83, et
c) les sondes ou les amorces d'au moins 14 nucléotides consécutifs, situées entre les positions 1261 et 2055 ou 1351 et 2055 de la séquence SEQ ID NO : 84.

4. Protéine isolée, **caractérisée en ce qu'**elle est codée par le polynucléotide selon la revendication 1 et que sa séquence est située des positions 3 à 540 de la séquence SEQ ID NO : 14.

5. Peptide, **caractérisé en ce qu'**il est sélectionné dans le groupe constitué par les peptides situés des positions : 293 à 540 et 323 à 540 de la séquence SEQ ID NO : 14.

6. Vecteur d'expression comprenant l'une des séquences définies aux revendications 1 et 3(a).

7. Vecteur d'expression, **caractérisé en ce qu'**il code pour au moins un peptide selon la revendication 5.

8. Animal transgénique non-humain, **caractérisé en ce qu'**il comprend un polynucléotide selon la revendication 1.

9. Anticorps, **caractérisé en ce qu'**il est dirigé spécifiquement contre un peptide selon la revendication 5.

10. Polynucléotide de séquence sélectionnée dans le groupe constitué par les séquences telles que définies à l'une quelconque des revendications 1 à 3 et les séquences complémentaires des séquences précédentes, pour utilisation comme réactif de diagnostic.

11. Anticorps selon la revendication 9 pour utilisation comme réactif de diagnostic.

12. Utilisation d'au moins un anticorps selon la revendication 9 pour la préparation d'un réactif de diagnostic d'une neuropathologie humaine à composante autoimmune.

13. Utilisation selon la revendication 12, **caractérisée en ce que** ladite pathologie est la sclérose en plaques.

14. Procédé de diagnostic d'une neuropathologie humaine à composante autoimmune, à partir d'un échantillon biologique, lequel procédé est **caractérisé en ce qu'**il comprend l'analyse de l'expression du transcrit tel que défini à la revendication 2 ou de la protéine selon la revendication 4.

15. Procédé selon la revendication 14, **caractérisé en ce que** ladite analyse est une analyse comparative entre un échantillon provenant d'un patient et un échantillon d'un individu normal servant de référence.

16. Procédé selon la revendication 14 ou la revendication 15, **caractérisé en ce qu'**il comprend :
(a) une étape dans laquelle l'on met en contact ledit échantillon biologique contenant des séquences nucléiques et/ou protéiques à analyser, avec au moins un réactif de diagnostic tel que défini à la revendication 10 ou 11, et
(b) une étape dans laquelle on détecte par tout moyen approprié les produits résultants de l'interaction entre lesdites séquences nucléiques ou protéiques et ledit réactif de diagnostic.

17. Procédé selon la revendication 16, **caractérisé en ce que** l'étape b) de détection comprend l'hybridation avec une sonde selon la revendication 3.

18. Procédé selon la revendication 16 ou 17, **caractérisé en ce que** le réactif de l'étape a) est un réactif nucléotidique tel que défini à la revendication 10, préalablement déposé sur un support approprié.

19. Procédé selon la revendication 16 ou 17, **caractérisé en ce qu'**il comprend :
* préalablement à l'étape (a) :
une étape de préparation du tissu ou du liquide biologique concerné, et
. une étape d'extraction de l'acide nucléique,
* préalablement à l'étape b) :
l'amplification dudit acide nucléique, à l'aide d'au moins une amorce selon la revendication 3 et
* postérieurement à l'étape (b) :
une étape de comparaison des séquences nucléiques amplifiées avec la séquence SEQ ID NO : 1.

20. Procédé selon la revendication 16, **caractérisé en ce que** l'étape b) comprend la mise en contact dudit échantillon biologique avec un anticorps selon la revendication 9, et la détection des complexes immunologiques formés, par tout moyen approprié.

21. Kit de diagnostic d'une neuropathologie humaine à composante autoimmune, **caractérisé en ce qu'**il comprend un réactif de diagnostic tel que défini à la revendication 10 ou 11.

## Claims

1. Isolated polynucleotide of sequence SEQ ID NO:1, corresponding to the *env* gene of an endogenous human retrovirus named HERV-7q.

2. Polyribonucleotide which is obtainable by transcription of the sequence as defined in claim 1.

3. Probe or primer for detecting endogenous human retroviral sequences of the family HERV-7q, **characterised in that** it is specific to the *env* gene and is selected from the group consisting of:
a) the probe of 2529 nucleotides located at positions 7053 to 9581 of the sequence SEQ ID NO: 2, capable of being amplified by the pair of primers SEQ ID NO:15 and SEQ ID NO:16,
b) the probes or primers having the sequences SEQ ID NO:15 to 27, 28-29, 82-83, and
c) the probes or primers of at least 14 consecutive nucleotides, located between positions 1261 and 2055 or 1351 and 2055 of the sequence SEQ ID NO:84.

4. Isolated protein, **characterised in that** it is coded by the polynucleotide according to claim 1 and its sequence is located at positions 3 to 540 of the sequence SEQ ID NO:14.

5. Peptide, **characterised in that** it is selected from the group consisting of the peptides located at positions 293 to 540 and 323 to 540 of the sequence SEQ ID NO:14.

6. Expression vector comprising one of the sequences defined in claims 1 and 3(a).

7. Expression vector, **characterised in that** it codes for at least one peptide according to claim 5.

8. Transgenic non-human animal, **characterised in that** it comprises a polynucleotide according to claim 1.

9. Antibody, **characterised in that** it is directly specifically against a peptide according to claim 5.

10. Polynucleotide having a sequence selected from among the sequences as defined in any one of claims 1 to 3 and the complementary sequences to the preceding sequences, for use as a diagnostic reagent.

11. Antibody according to claim 9 for use as a diagnostic reagent.

12. Use of at least one antibody according to claim 9 for preparing a reagent for diagnosing a human neuropathology with an autoimmune component.

13. Use according to claim 12, **characterised in that** said pathology is multiple sclerosis.

14. Process for diagnosing a human neuropathology with an autoimmune component, from a biological sample, said process being **characterised in that** it comprises analysing the expression of the transcript as defined in claim 2 or the protein according to claim 4.

15. Process according to claim 14, **characterised in that** the analysis is a comparative analysis between a sample taken from a patient and a sample from a normal individual serving as a reference.

16. Process according to claim 14 or claim 15, **characterised in that** it comprises:
(a) a step wherein said biological sample containing nucleic and/or protein sequences to be analysed is brought into contact with at least one diagnostic reagent as defined in claim 10 or 11, and
(b) a step wherein the products resulting from the interaction between said nucleic and/or protein sequences and said diagnostic reagent are detected by any suitable means.

17. Process according to claim 16, **characterised in that** the detection step b) comprises hybridisation with a probe according to claim 3.

18. Process according to claim 16 or 17, **characterised in that** the reagent in step a) is a nucleotide reagent as defined in claim 10, placed beforehand on a suitable support.

19. Process according to claim 16 or 17, **characterised in that** it comprises;
- prior to step (a):
a step of preparing the tissue or the biological liquid in question, and
a step of extracting the nucleic acid,
- prior to step (b):
amplification of said nucleic acid, using at least one primer according to claim 3, and
- after step (b):
a step of comparing the amplified nucleic sequences with the sequence SEQ ID NO:1.

20. Process according to claim 16, **characterised in that** step b) comprises bringing said biological sample into contact with an antibody according to claim 9, and detecting the immunological complexes formed, by any suitable method.

21. Diagnostic kit for a human neuropathology with an autoimmune component, **characterised in that** it comprises a diagnostic reagent as defined in claim 10 or 11,

## Patentansprüche

1. Polynucleotid, isoliert aus der Sequenz SEQ ID NR. 1, entsprechend dem *env-*Gen eines HERV-7q genannten endogenen menschlichen Retrovirus.

2. Polyribonucleotid, erhältlich durch Transkription der nach Anspruch 1 definierten Sequenz.

3. Sonde oder Primer für die Detektion retroviraler endogener menschlicher Sequenzen der HERV-7q-Familie, **dadurch gekennzeichnet, dass** sie/er für das *env-*Gen spezifisch ist und ausgewählt ist aus der Gruppe, bestehend aus:
a) einer Sonde von 2529 Nucleotiden der SEQ ID NR. 2, die sich in den in den Positionen 7053 bis 9581 befindet; erhältlich durch Amplifizieren des Primerpaars SEQ ID NR. 15 und SEQ ID NR. 16,
b) die Sequenzen SEQ ID NR. 15 bis 27, 28-29, 82-83 aufweisende Sonden oder Primer und
c) Sonden oder Primer wenigstens 14 aufeinanderfolgender Nucleotide, die sich zwischen den Positionen 1261 und 2055 oder 1351 und 2055 der Sequenz SEQ TD NR 84 befinden.

4. Isoliertes Protein, **dadurch gekennzeichnet, dass** es von dem Polynucleotid nach Anspruch 1 codiert wird und dass sich seine Sequenz in den Positionen 3 bis 540 der Sequenz SEQ ID NR. 14 befindet.

5. Peptid, **dadurch gekennzeichnet, dass** es ausgewählt ist aus der Gruppe, bestehend aus den Peptiden in den Positionen 293 bis 540 und 323 bis 540 der Sequenz SEQ ID NR. 14.

6. Expessionsvektor umfassend eine der nach den Ansprüchen 1 und 3(a) definierten Sequenzen.

7. Expressionsvektor, **dadurch gekennzeichnet, dass** er für wenigstens ein Peptid nach Anspruch 5 codiert.

8. Nicht-menschliches transgenes Tier, **dadurch gekennzeichnet, dass** es ein Polyribonucleotid nach Anspruch 1 enthält.

9. Antikörper, **dadurch gekennzeichnet, dass** er spezifisch gegen ein Peptid nach Anspruch 5 gerichtet ist.

10. Polynucleotid einer Sequenz, ausgewählt aus der Gruppe, bestehend aus den nach einem der Ansprüche 1 bis 3 definierten Sequenzen und komplementären Sequenzen der vorhergehenden Sequenzen, zur Verwendung als diagnostisches Reaktionsmittel.

11. Antikörper nach Anspruch 9 zur Verwendung als diagnostisches Reaktionsmittel.

12. Verwendung wenigstens eines Antikörpers nach Anspruch 9 zum Herstellen eines diagnostischen Reaktionsmittels zur Diagnose einer menschlichen neuropathologischen Krankheit mit Autoimmunkomponente.

13. Verwendung nach Anspruch 12, **dadurch gekennzeichnet, dass** die Krankheit Multiple Sklerose ist.

14. Diagnoseverfahren einer menschlichen neuropathologischen Krankheit mit Autoimmunkomponente, ausgehend von einer biologischen Probe, wobei das Verfahren **dadurch gekennzeichnet ist, dass** es eine Analyse des nach Anspruch 2 definierten Transkripts oder des nach Anspruch 4 definierten Proteins umfasst.

15. Verfahren nach Anspruch 14, **dadurch gekennzeichnet, dass** die Analyse eine Vergleichsanalyse zwischen einer von einem Patienten stammenden Probe und einer, als Referenz dienenden, von einem normalen Individuum stammenden Probe ist.

16. Verfahren nach Anspruch 14 oder 15, **dadurch gekennzeichnet, dass** es umfasst:
(a) einen Schritt in dem die die zu analysierenden Nukleinsequenzen und/oder Proteinsequenzen enthaltende biologische Probe mit wenigstens einem diagnostischen, nach den Ansprüchen 10 oder 11 definierten Reaktionsmittel in Kontakt gebracht wird, und
(b) einen Schritt, in dem mit allen geeigneten Mitteln die resultierenden Produkte der Wechselwirkung zwischen den Nukleinsequenzen oder Proteinsequenzen und dem diagnostischen Reaktionsmittel detektiert werden.

17. Verfahren nach Anspruch 16, **dadurch gekennzeichnet, dass** der Detektionsschritt b) die Hybridisierung mit einer Sonde nach Anspruch 3 umfasst.

18. Verfahren nach Anspruch 16 oder 17, **dadurch gekennzeichnet, dass** das Reaktionsmittel im Schritt a) ein nach Anspruch 10 definiertes nukleotidhaltiges Reaktionsmittel ist, das vorab auf einem geeigneten Träger abgeschieden wurde.

19. Verfahren nach Anspruch 16 oder 17, **dadurch gekennzeichnet, dass** es umfasst:
* vor dem Schritt (a):
• einen Schritt des Herstellens eines betreffenden Gewebes oder einer betreffenden biologischen Flüssigkeit, und
• einen Schritt der Extraktion einer Nukleinsäure
* vor dem Schritt (b);
Amplifizierung der Nukleinsäure mit Hilfe wenigstens eines Primers nach Annspruch 3 und
* nach dem Schritt (b):
einen Schritt des Vergleichs der amplifizierten Nukleinsequenzen mit der SEQ ID NR. 1.

20. Verfahren nach Anspruch 16, **dadurch gekennzeichnet, dass** der Schritt b) das in Kontakt bringen der biologischen Probe mit einem Antikörper nach Anspruch 9 und die Detektion des gebildeten immunologischen Komplexes mit jedem geeigneten Mittel umfasst.

21. Diagnosekit für eine menschlichen neuropathologische Krankheit mit Autoimmunkomponente, **dadurch gekennzeichnet, dass** er ein nach Anspruch 10 oder 11 definiertes diagnostisches Reaktionsmittel umfasst.
